# EUROPEAN PATENT SPECIFICATION

(11) **EP 2 219 033 B1**
(45) Date of publication and mention of the grant of the patent: **25.05.2016**
(21) Application number: 08022560.0
(22) Date of filing: 30.12.2008
(51) Int. Cl.: G01N 33/543, G01N 35/00

(54) **Device, instrument and process for detecting magnetically labeled analytes**
Vorrichtung, Instrument und Verfahren zur Erkennung von magnetisch etikettierten Analyten
Dispositif, instrument et procédé de détection des analytes étiquetés magnétiquement

(43) Date of publication of application: 18.08.2010
(73) Proprietor: MicroCoat Biotechnologie GmbH, 82347 Bernried (DE); Igedi GbR, 76149 Karlsruhe (DE); ACI AG, 78658 Zimmern ob Rottweil (DE); BIT Analytical Instruments GmbH, 65824 Schwalbach (DE); Ernst Reiner GmbH & Co. KG, 78120 Furtwangen (DE); Sensitec GmbH, 36633 Lahnau-Waldgirmes (DE); i-sys Mikro- und Feinwerktechnik GmbH, 76229 Karlsruhe (DE); Universität Bielefeld, 33615 Bielefeld (DE)
(72) Inventor: Waltenberger, Harald, Dr., 85737 Ismaning (DE); Müller, Günter, Dr., 82347 Bernried (DE); Gengenbach, Ulrich, Dr., 75196 Remchingen (DE); Dickerhof, Markus, Dr., 76149 Karlsruhe (DE); Schmitt, Jochen, 35216 Biedenkopf (DE); Loreit, Uwe, 35580 Wetzlar (DE); Harter, Karl, 78120 Furtwangen (DE); Lorenz, Michael, 79211 Denzlingen (DE); Glathe, Andreas, Dr., 69469 Weinheim (DE); Seemann, Herbert, 78052 Villingen-Schwenningen (DE); Gentischer, Josef, 71384 Weiterstadt-Beutelsbach (DE); Hütten, Andreas, Prof. Dr., 33609 Bielefeld (DE); Brenk, Uwe, 75236 Kämpfelbach (DE)
(74) Representative: Grünecker Patent- und Rechtsanwälte PartG mbB

(56) References cited:
- WO-A-2006/119308
- US-A1- 2008 160 622

## Description

### Field of the invention

The present invention is directed to a device for quantitative analysis of an analyte in a liquid sample by detecting a magnetic label, an instrument for controlling the analysis process and displaying the results and a method for performing said analysis with said device and said instrument.

### State of the art

The use of different solid materials (e.g. magnetic particles, micro plates, tubes) to bind immunological complexes (e.g. ELISA, ELIA, CLEIA, EIA, RIA) and to detect light or fluorescence to measure a specific analyte are well known and described in a lot of patents and papers. Primary for human diagnostics closed systems like Elecsys (Roche Diagnostics GmbH), Centaur (Bayer AG), Architect (Abbott Inc.), VIDA/VIDAS (Biomerieux) are distributed world wide in clinical labs or lab organizations for medium or high throughput measurement of samples (>600 samples a day). Also a lot of smaller open systems exist for measurement of the same assay panels based on micro plates. Dependent on the detection technologies the systems are expensive and educated personal is needed to run the systems moreover they are usable only stationary.
Other technologies as lateral flow (test strips) are available. They are used for single sample measurements in areas such as personalized diagnostics. The detection is mostly based on the color or color change of gold particles but the test principles are the same as mentioned above. The mobile use is the big advantage but most of them give only qualitative results and not all tests are applicable.
Especially the personalized medical treatment in combination with the personalized diagnostics will change the markets in the near future. For this field of human diagnostics small and flexible systems e.g. for doctor's office, clinical labs or patients have to be available. In addition such systems are usable in all fields of diagnostics like food, veterinary, drug of abuse and others.
Some new patents describe methods to use magnetic particles and detect the change of a magnetic field used for specific analyte detection.
EP Apl. No 04 772 339.0 *(Biosensor and Method of Assaying Object)* describes a sensor for magnetic particle detection. This sensor is defined as a device with a silicon nitride or silicon oxide surface, specially activated for selective immobilization. Above the sensor is a reagent chamber arranged and the sensor is defined as a Hall sensor. The magnetic particles and analyte will be dispensed inside the chamber followed by a magnetic mixing. The magnetic particles will be attracted onto the sensor surface with a magnetic field. A magnetic washing step helps to discriminate between bound and non bound magnetic particles. This technology is not usable for a handheld due to the high energy requirements for the magnet particle mixing, attraction to surface, "washing" and detection. The main focus of this application is on magnetic sensor optimization and sensor signal/data processing. WO 2007/060568 describes a magnetic sensor device encompassing a sample chamber for providing a magnetically interactive sample, magnetic field generators and a magnetic sensor element.
US 2006/0081954 A1 decribes a magnetic field detection element, including a channel structure for guiding a sample across said detection element. That device is fabricated by thin film processes.
In the state of the art the devices are usually built by using the same thin film processes that were used to fabricate the magnetic sensor element to also fabricate the respective fluidic channel structure. This makes such devices expensive.
In WO 02/067004 A1 and US 6,597,176 B2 (Quantum Design) the detection of the magnetic field is state of the art. In particular focus of the descriptions is the unit to detect the magnetic beads. The method is a quantitative one. Quantum Design is using a solid substrate where the ligand magnetic particle complexes accumulate in a spatial pattern (lateral flow membranes). They are using an equipment to scan the solid substrate where the particles are bound in the spatial pattern. The equipment to scan the solid substrate (Hall sensor) is a part of the detection unit and not suitable for being integrated in a disposable. The use of a DC magnetic field in combination with the Hall sensor needs a high power to generate the field and the limitation is the gap between the solid substrate and the field generator. This yields a low sensitivity. There is also no clear description of the discrimination of bound and non bound magnetic particles.
The patents by Quantum Design describe a complete system comprising device, instrument and a corresponding detection method. This approach is, however, very limited with respect to detection limit, does not encompass sample preprocessing and does not lend itself to implementation in small mobile instruments with disposable devices.

US 2008/160622 discloses a device for detecting an analyte in the sample. An array of magnetic microcoils is employed to generate a magnetic field. The magnetic field is used for particle transfer and separation. The detection unit of the signal indicating the presence of an analyte is a sensor detecting optical or electrical signals.

Accordingly, it is one object underlying the present invention to provide a detection means, which overcomes these disadvantages of the patents by Quantum Design.

There is in particular a need in the art for a means that covers the entire process chain from sample preparation to detection.

Accordingly, it is one object underlying the present invention to provide such a means.

There is also a need for the implementation of a magnetic label detecting system means into a disposable device, for a corresponding instrument employing the disposable device and for processes to fabricate the device in an efficient and economic way.

Accordingly, it is another object underlying the present invention to provide such a detecting system (instrument), a disposable device and processes to fabricate the device.

There is also a need for increasing the sensitivity of the detector for magnetic labels.

Accordingly, it is another object underlying the present invention to provide a detection system with an increased detection sensitivity compared to the patents by Quantum Design.

The fabrication technology for a disposable is an important issue to bring such a device to the market in high quantity, good quality and at low cost.
Accordingly, it is another object underlying the present invention to provide highly economic and low cost fabrication process for a disposable.

### Summary of the invention

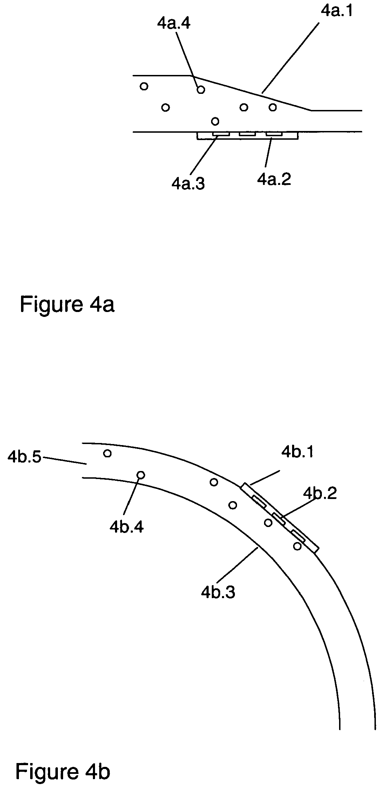

To solve one or more of these objectives, the present invention provides a device for detecting an analyte bound to one or more magnetic particle labels, preferably in a sample, the device comprising
A) at least one fluidic channel structure for transporting and processing magnetic particles, and
B) an integrated magnetic sensor element, wherein
   the magnetic sensor element is part of at least one channel wall, and the fluid channel geometry is optimized for fast and uniform binding of magnetic particle/analyte complexes on the sensor surface, by leading fluid flow over the sensor surface wherein the magnetic particle/analyte complexes are guided onto the surface of the magnetic sensor element by E1) an alteration of the fluidic flow profile such as by a gradual reduction of channel height (fig. 4a.1) over the sensor or E2) by inertial forces generated by guiding the flow in a curved channel (fig. 4b.3) wherein the sensor element is positioned in a manner that it can be targeted by the flow, e.g. in the outer channel wall in the curve and the magnetic sensor element is a GMR, TMR, CMR, EMR or Hall-sensor.

Guiding the sample flow over the magnetic sensor surface of this device increases the binding efficiency on the sensor surface and therewith the detection sensitivity.

In a preferred embodiment, the device allows for an "holistic approach" as it is suitable for being used in a detection method including the steps of
- sample insertion into a disposable device,
- integrated sample preprocessing,
- storing of magnetic particles in the device
- controlled release of stored magnetic particles
- controlled binding of analyte to the particles
- guiding the sample flow over a magnetic sensor element to increase binding efficiency on the sensor surface and
- removing unspecifically bound particles.

The device may be designed as a disposable device and may be fabricated in an efficient and economic way.

The invention disclosed here achieves the above technical benefits by applying low cost mass fabrication processes such as injection molding. This opens new mass markets to this detection method.

In summary, the invention encompasses a device for quantitative analysis of an analyte in a sample, preferably in a liquid sample, an instrument for controlling the analysis process and displaying the results and a method for performing said analysis with said device and said instrument as well as a fabrication process of the device.

### Detailed Description of the invention

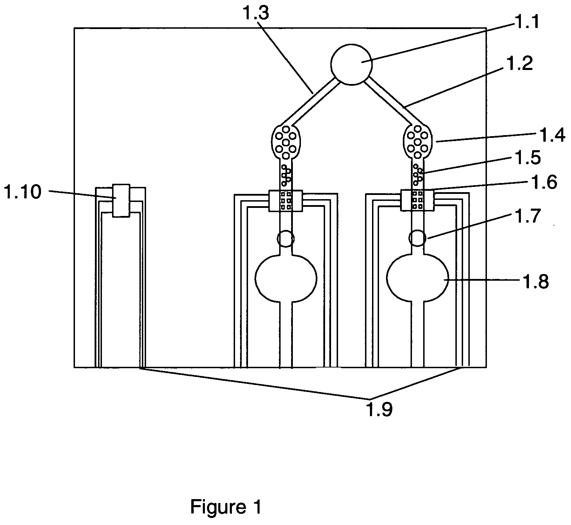

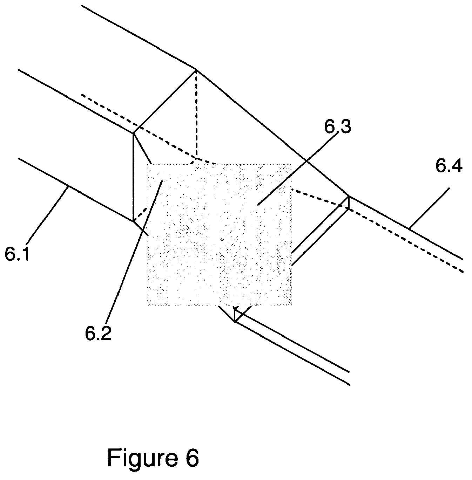

In a first aspect, the present invention provides a device for detecting an analyte bound to one or more magnetic particle labels, preferably in a sample, the device characterized by A ) at least one fluidic channel structure for transporting and processing magnetic particles, and B) an integrated magnetic sensor element, wherein the magnetic sensor element is part of at least one channel wall, and/or the fluid channel structure geometry is optimized for fast and uniform binding of magnetic particle/analyte complexes on the sensor surface, by leading fluid flow over the sensor surface wherein the magnetic particle/analyte complexes are guided onto the surface of the magnetic sensor element by E1) an alteration of the fluidic flow profile such as by a gradual reduction of channel height (fig. 4a.1) over the sensor or E2) by inertial forces generated by guiding the flow in a curved channel (fig. 4b.3) wherein the sensor element is positioned in a manner that it can be targeted by the flow, e.g. in the outer channel wall in the curve and the magnetic sensor element is a GMR, TMR, CMR, EMR or Hall-sensor.
A first illustrative embodiment of this device 1,4 and 6 is described along the figures of the present specification as follows: In the device (Figure 1) for detecting an analyte bound to one or more magnetic particle labels an analyte, which is preferably in a sample, is guided through at least one fluidic channel structure (Figure 1.2) onto an integrated magnetic sensor element (Figure 1.6).
The magnetic sensor element is part of at least one channel wall, and/or the fluid channel geometry is optimized for fast and uniform binding of magnetic particle/analyte complexes on the sensor surface (Figure 4a, 4b), by leading fluid flow over the sensor surface.
In order to guide the particles to the sensor surface, the fluid channel geometry is optimized for fast and uniform binding of magnetic particle/analyte complexes on the sensor surface, by leading fluid flow over the sensor surface (Figure 4a, 4b).
For example, the channel ceiling height is gradually decreased, while channel width increases to maintain a constant cross section and thus flow (Figure 6). In this way the probability that the analyte molecules bound to magnetic particles bind to second receptor is increased.
Fig 1 shows a second illustrative embodiment of the device of the present invention. The orifice (1.1) for insertion of the sample leads via an analysis channel structure (1.2), a chamber (1.4) for storage of magnetic particles and a means for mixing (1.5) to the magnetic sensor element (1.6). Behind the magnetic sensor element follows a magnetic field generation element (1.7) and a waste chamber (1.8). A reference channel structure (1.3) of identical layout runs parallel to the analysis channel structure (1.2). Conductors (1.9) allow connecting the magnetic sensor elements (1.6) and the identification/memory element (1.10) to the instrument.

In a first preferred embodiment of the device according to the first aspect of the present invention, the device of the first aspect further comprises a means for optimizing dissolution of magnetic particles flowing through the channel structure. Here, the magnetic particles, which may or may not be coated with a the first receptorfirst receptor and/or analyte are released from the means for optimizing dissolution of magnetic particles. The means is a reservoir of said coated or uncoated magnetic particles and is suitable to release the particles into the sample flow of the channel structure.

The means may form (part of) a container or chamber harbouring the coated or uncoated magnetic particles.

The means may comprise the coated or uncoated magnetic particles embedded in a matrix, which matrix may be soluble in the sample. Examples for the material of the matrix are saccharose, glycerol, PEG, proteins, or their combinations among others.

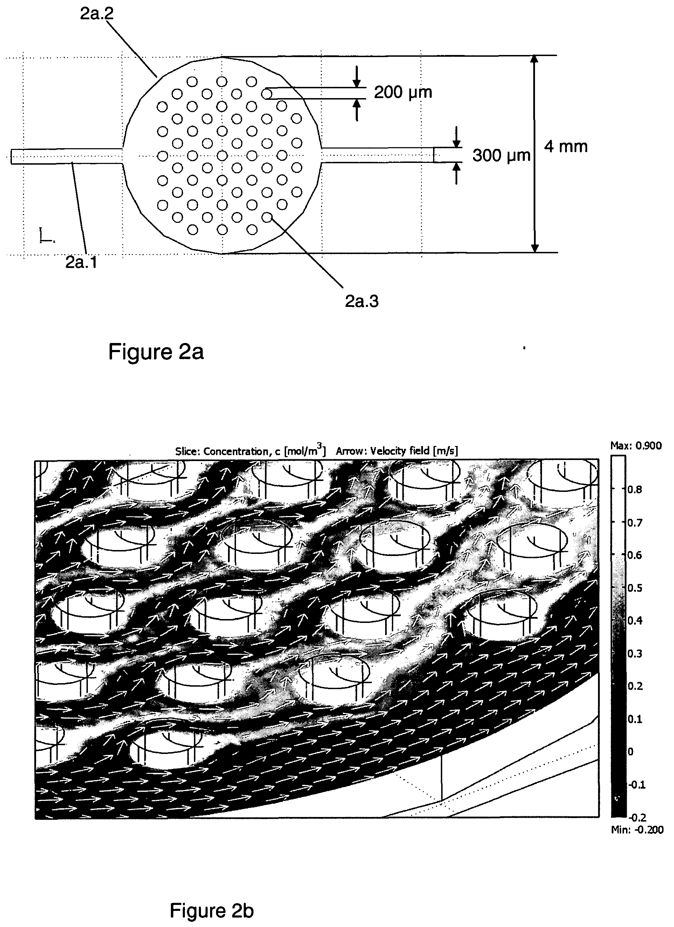

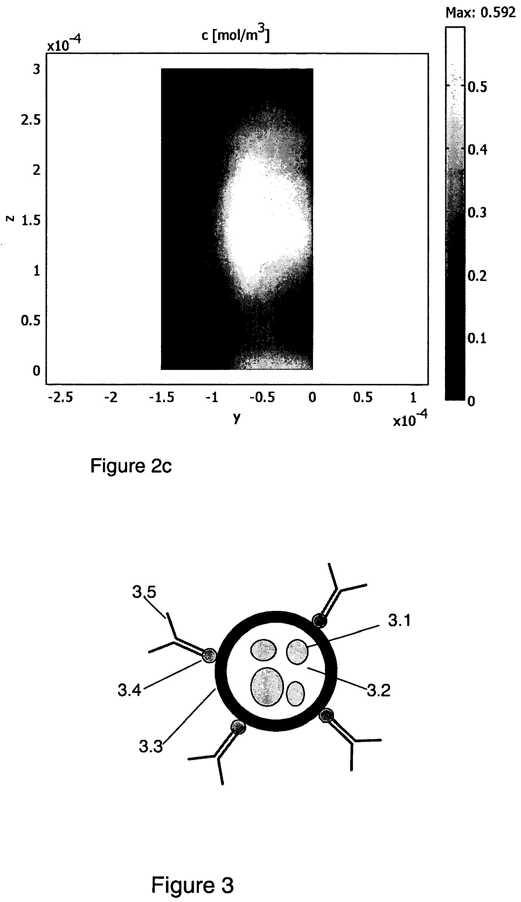

The means may form (part of) a container or chamber which is part of the channel structure. The means preferably forms one or more separate chambers, which are structured in such a way that the particles are released at different heights in the flow. Preferably, the means may form columns of different heights (see Figure 2).

In one embodiment the means is a part of the channel structure comprising magnetic particles which are coated in a thin layer onto the channel walls and/or ceiling.

In another embodiment, the dissolution can be maximised, wherein the means is a chamber with columns of different lengths coming down from the channel structure ceiling. The layer of magnetic particles may now be distributed over a much larger surface area with increased contact with the sample flow.

The shape, length and spacing of the columns can easily be optimised for uniform flow resistance over the chamber cross section, thus achieving a very uniform dissolution of the magnetic particles across height and width of the channel.

Figure 2 shows a segment (2a.1) of the channel structure with a preferred embodiment of the means for dissolution of the magnetic particles. It consists of a chamber (2a.2) with columns (2a.3) on which the magnetic particles are coated.

In a second preferred embodiment of the device according to the first aspect of the present invention, the device further comprises magnetic particles coated with the first receptor, which are stored in the device and are capable of binding to the analyte, once a flow of the sample through the channel structure occurs.

The first receptor may be an antibody, a receptor molecule, a phage protein or DNA. Attaching the first receptor to magnetic particles is well known in the art.

Figure 3 shows a coated magnetic particle consisting of magnetic core particles (3.1) surrounded by a shell (3.2), whose surface is activated (3.3). A linker molecule (3.4) connects the first receptor (3.5) to the magnetic particle.

In a third preferred embodiment of the device according to the first aspect of the present invention, magnetic particle/analyte complexes are guided onto the surface of the magnetic sensor element
- by an alteration of the fluidic flow profile such as by a gradual reduction of channel height (fig 4a.1) over the sensor, preferably with constant cross sectional area, or
- by inertial forces generated by guiding the flow in a curved channel (see e.g. fig 4b.3) whereby the sensor element is positioned that it can be targeted by the flow, e.g. in the outer channel wall in the curve.

Simulation Experiments have shown that sedimentation of magnetic particles in the µm size range or below in a channel of 100 µm height takes up to several minutes (see Example 2). This is not compatible with a measuring principle that is intended to yield results in minutes. Hence, there are two proposals to accelerate sedimentation:
a) By gradually lowering the channel ceiling while the channel width is increased. In a preferred embodiment the channel height is lowered from e.g.100 µm to e.g. 10 µm. This forces the magnetic particles downwards onto the sensor surface and thus accelerates sedimentation time down to a few seconds.
b) By guiding the flow around a curve the particles are carried to the outer wall of the channel due to using centrifugal forces similar as e.g. in a mass spectrometer. If the sensor element is integrated in this outer wall the particles will settle on its surface.

Fig. 4 shows these two approaches. Figure 4a shows a segment of the channel structure with reduction of the channel height (4a.1) above the sensor element (4a.2), to accelerate sedimentation and thus binding of the magnetic particles (4a.4) on the sensor element surfaces (4a.3).
Figure 4b shows a curved segment (4b.3) of the channel structure (4.b.5), where magnetic particles (4b.4) get deflected due to inertia and land on the sensor element surfaces (4b.2) of the sensor element (4b.1).

Figure 5a shows results of sedimentation experiments with 1 µm sized magnetic particles. The x-axis units is sedimentation time in 10³ s, the y-axis shows the starting height of the sedimentation experiment in Millimetres.

Figure 5b shows simulation results of the channel structure with height reduction over the magnetic sensor element. The inlet height of the channel was 50 µm, its outlet height 5 µm. Different starting heights of the magnetic particles (x-axis) were assumed. The y-axis denotes the landing position measured from the entry of the height reduced channel part. The numbers at the data points indicate the sedimentation time in seconds.

Figure 6 shows the channel segment with the height reduction over the magnetic sensor element. The channel inlet (6.1) is reduced in height (6.2) above the magnetic sensor element (6.3) and widened to the outlet (6.4).

In a fourth preferred embodiment of the device according to the first aspect of the present invention, the device further comprises
an element for mixing sample and magnetic particles to facilitate binding or competition of analyte to particle by means of the first receptor, preferably in a channel or a chamber, wherein the element for mixing is
   ▪ a passive structure such as a change of channel cross section (e.g. columns) or direction (e.g. meander) or a combination of both; or
   ▪ an active structure such as a ferromagnetic element lying loosely in the channel structure or in a chamber that may be moved by an alternating magnetic field.

In this embodiment the analyte within a sample is introduced into the device to get into contact with the magnetic label particle(s). For mixing of sample and magnetic particles a mixing element can be integrated into the channel structure.

The magnetic particles, which are dissolved in the flow within the channel structure, and the sample get intensively mixed by an element for mixing sample and magnetic particles while further flowing through the channel structure. During this process the analyte contained in the sample binds with the first receptor and via binding to the the first receptor gets attached to the magnetic particles.

This can be realised passively by meanders or arrays of columns in the channel structure in the plane or three dimensionally. Since laminar flow dominates in these small dimensions these passive elements have the task to decrease diffusion lengths by dividing, folding and reuniting the flow.

Another option is to integrate an active element into the channel structure, such as a moveable ferromagnetic element, that is actuated linearly or rotatory like a miniaturised magnetic stirrer. This significantly improves mixing compared to the above passive structures.

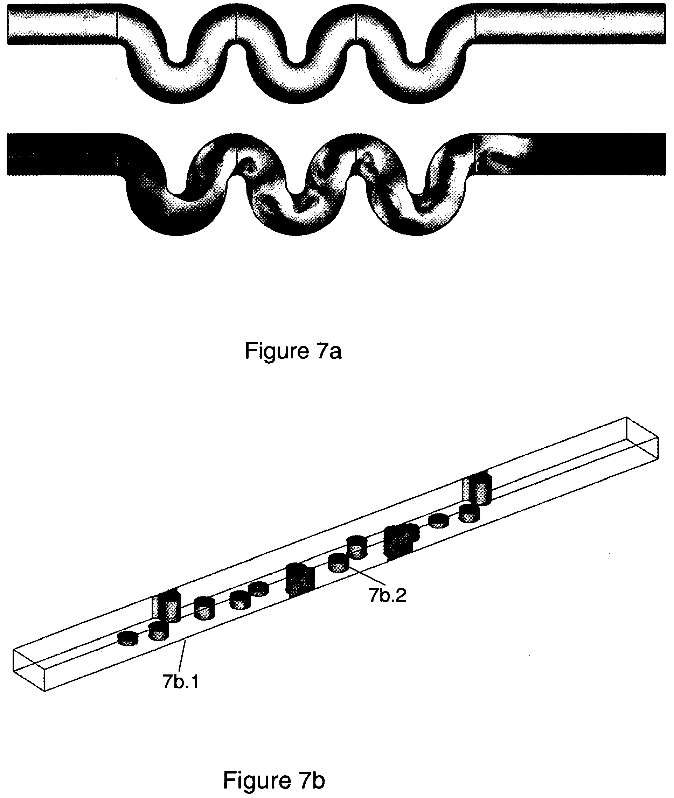

Figure 7a shows fluid dynamics simulations at different scales. In the lower image at a channel of 10 mm turbulent flow can be observed and thus intensive mixing be expected. In the upper image with channel width reduced to 1 mm all other variables remaining unchanged only laminar flow can be observed and thus poor mixing only due to Brownian motion can be expected.

Figure 7b shows a segment of the channel structure (7b.1), where columns of different height (7b.2) are distributed in order to improve laminar mixing by separating, folding and reuniting fluid flow.

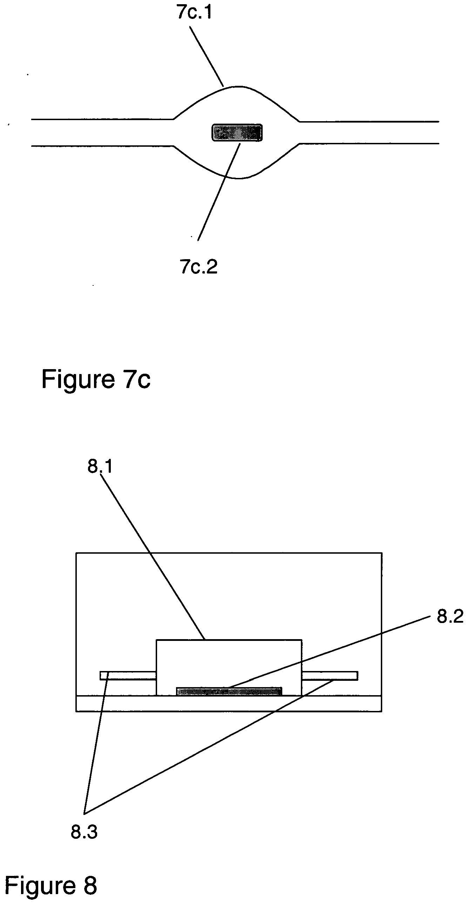

Figure 7c shows a segment of the channel structure with a chamber (7c.1) with a movable member (7c.2).

In the device according to the first aspect of the present invention, the magnetic sensor element is a GMR, TMR, CMR, EMR or a Hall-sensor and may comprise at least one sensor element surface or a linear or two dimensional array of sensor element surfaces.

The use of magneto resistive sensors for detection of magnetic particles is state of the art as documented in patents by Motorola (US6057167A), Philips (WO03054566A1) and NVE (US020070236212A1).

The sensor element can consist of a single sensor element surface or of a linear or two-dimensional sensor element surface array. A single sensor surface allows only measurement of one analyte. Several sensor element surfaces can be used to measure the same analyte with more than one sensor element surface, but also for parallel detection of a number of different analytes in the sample.

In a sixth preferred embodiment of the device according to the first aspect of the present invention, at least one ferromagnetic element is integrated as a magnetic flux concentrator in the vicinity of, preferably besides, the sensor element.

In order to maximize the magnetic field around the magnetic sensor ferromagnetic elements can be positioned in its vicinity. These can be located in the instrument or insert molded in the device. In the latter case they are positioned in the channel walls adjacent to the magnetic sensor. They may be entirely embedded in the device or reach up to the device surface in order to get into direct magnetic contact with pole shoes in the instrument.
Figure 8 schematically shows a cross section of the device where ferromagnetic elements (8.3) are integrated to the left and to the right of the channel structure (8.1) in order to achieve magnetic field concentration over the magnetic sensor element (8.2).
In a seventh preferred embodiment of the device according to the first aspect of the present invention, the magnetic sensor surface is coated with gold to allow the specific binding of a second receptor. The second receptor may be an antibody, a receptor molecule, a phage protein, DNA. Optionally the second receptor may comprise a thiol-linker suitable for gold binding. After getting further carried along in the channel structure by the flow, the particles arrive over the magnetic sensor element that is embedded in the floor of the channel. In the seventh embodiment the surface of the magnetic sensor element is coated with the second receptor. To allow binding of the second receptor, the surface of the sensor element may be coated with gold.

The specific coating of gold sole (i.e. gold nano particles) is a well-known technology to be used for lateral flow technologies and usable for other gold surfaces like the sensor surface. Without activation the binding of proteins is possible. Improvements can be achieved by implementation of sulfur groups at protein sites. The gold surface is coated with the specific reagents by e.g.
a) direct (adsorptive) coating of the specific reagent ( second receptor the second receptor)
b) or on modular basics like pre coating with e.g. Streptavidin/Biotin or anti-IgG (also adsorptive or chemical) and the second coating of the specific reagent ( the second receptor).

Preferably, surrounding surfaces, which are not gold coated for binding to the second receptor, are coated with a different material (e.g. Al₂O₃, SiO₂, SiN or others), which is inert for binding of the first receptor, the second receptor or analyte.

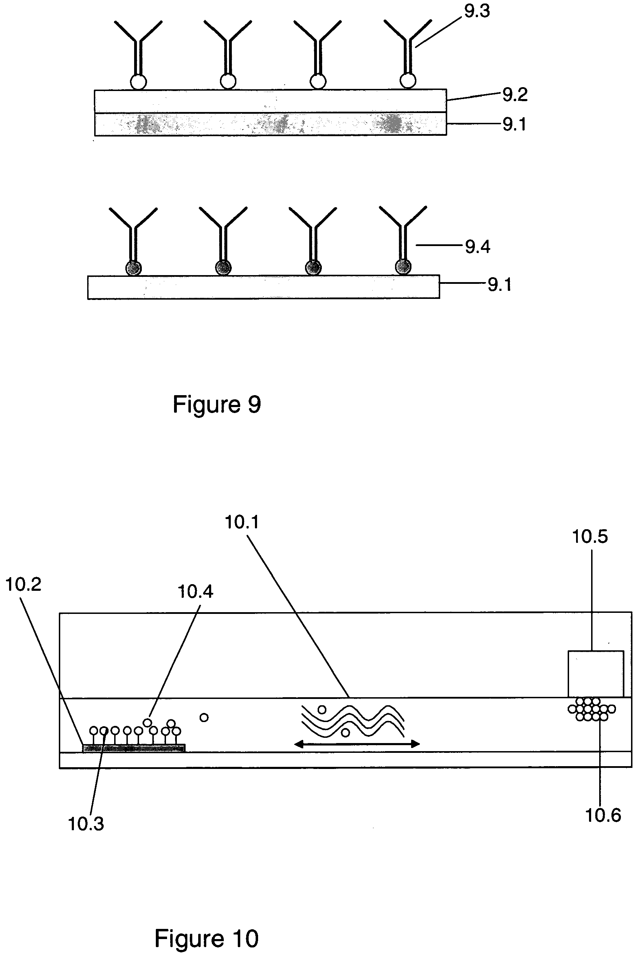

Figure 9 schematically shows two variants of the coating of a sensor element surface. In one case a) an activated second receptor (e.g. sulphur activated) is directly bound on a gold layer (9.1). Another option is a surface activation (9.2) e.g, streptavidin on which second receptor (9.3) is bound with the biotin streptavidin bond.
In an eighth preferred embodiment of the device according to the first aspect of the present invention, a fluid flow in the channel structure is achieved by either capillary forces, by electro wetting or by a pumping element integrated in the device or by attaching said device to an external pumping element.

There are several ways to achieve fluid flow in micro fluidic channels:
▪ Capillary forces are utilised e.g. in CE-chips.
▪ Electro wetting as technology to transport fluids in micro fluidic channels is state of the art see e.g. Pollack, M., Fair, R., Shenderov, A., 2000, "Electro wetting-based actuation of liquid droplets for micro fluidic applications," Appl. Phys. Lett., vol.77, p.1725.]
▪ In a device integrated pumping elements realised by a chamber with a bottom, top or wall made of an elastic membrane that is deformed from the outside by means of an actuator.
▪ A micro pump (e.g. mp5 by Bartels Mikrotechnik) can be integrated in the instrument and is connected to the device upon insertion.

In a ninth preferred embodiment of the device according to the first aspect of the present invention, an element to generate a magnetic field (e.g. permanent magnet, coil) is integrated downstream from the magnetic sensor element in the vicinity of the channel structure to capture unbound particles.

It may happen that particles that are not specifically bound to the second receptor remain lying loosely on the sensor element surface. Since these particles would distort the measurement, in a preferred embodiment, they are removed from the sensor. One means to achieve this is using a magnetic field generator (e.g. a permanent magnet) to capture unbound particles. This magnetic field generator can be either integrated in the device or in the instrument (see claim 10)

The magnetic field generator (e.g. a permanent magnet) is located a short distance downstream from the sensor above the channel. The distance (e.g. 10 mm) is chosen such that the magnetic field does not interfere with the sensor element. By means of the flow the sample is moved in the channel structure from the sensor to the place where the magnetic field generator is located. The shear flow resulting from the pumping removes the loose magnetic particles from the sensor surface and transports them in the vicinity of the magnet. Here they are captured by the magnetic field gradient and remain attached to the channel wall when the sample is being moved the next time. That way the fluid is being purified from the magnetic particles and can be pumped back over the sensor surface without transporting particles back. The means may be applied several times in order to bit by bit remove all unbound particles from the sensor surface.

The benefit of this approach is that this washing is achieved by reusing the same small fluid volume every time this is an important advantage for running such a process in a device.

At the end of the fluid channel excess fluid material may be collected in a waste chamber that is equipped with wicking membrane to avoid flow of fluid into the instrument which would yield contamination of the pump.

Figure 10 shows a segment of the channel structure (10.1) with the magnetic sensor element (10.2) with bound magnetic particles (10.3) and unbound magnetic particles (10.4). An alternating flow (10.1) removes the unbound magnetic particles and transports them to the magnetic field generator (10.5) where they are held (10.6) by magnetic forces.

In a tenth preferred embodiment of the device according to the first aspect of the present invention, a means for sample pre-treatment (e.g. filter and/or reagent) is integrated upstream from the magnetic sensor element.

Sample pretreatment can be done
▪ mechanically e.g. by filtering such as blood plasma separation by filter membranes (e.g. products by Pall, Whatman) or
▪ chemically e.g. prevention of coagulation by pre deposition of reagents like heparin or citrate.
▪ or combinations of both

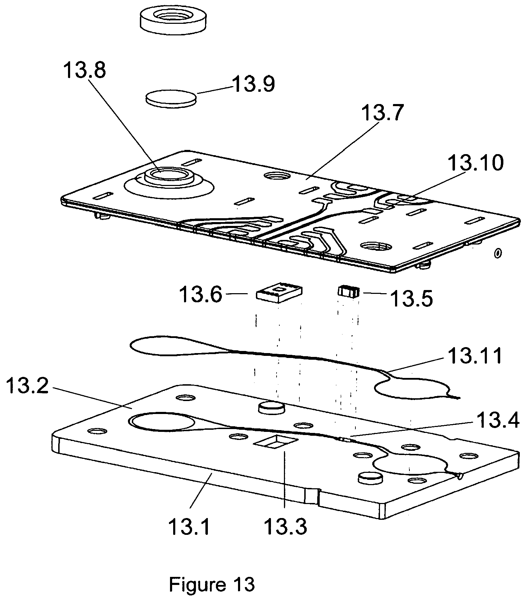

This is illustrated schematically in Figure 1 and in a preferred embodiment in Figure 13.

In an eleventh preferred embodiment of the device according to the first aspect of the present invention, the device further comprises an inlet of the channel structure, wherein the sample is being applied at the inlet of the channel structure whereby the inlet is an opening, e.g. an element to directly take over blood from the finger or e.g. an element to accommodate a tube, pipette, syringe.

The device can be equipped with orifices for direct transfer of blood from the finger or with standard interface to attach tubes syringes etc. such as swage-lock, Luer-Lock.

This is illustrated schematically in Figure 1 and in a preferred embodiment in Figure 13.

In a twelfth preferred embodiment of the device according to the first aspect of the present invention, the device further comprises a reference channel structure including an integrated magnetic sensor element coated with second receptor, and a defined amount of analyte bound or not bound to magnetic particles pre-coated with the first receptor sufficient to verify the kinetics of either a direct or a competitive binding.

To verify the function of the used reagents, controls (of analyte) may be measured. In analyzer systems like Elecsys, Centaur or Architect control reagents are measured within a run in between samples. With the technology used here the control of the analyte can be done by an internal control or in parallel with a reference channel if necessary. The reference channel differs from the analysis channel in that it contains magnetic particles with the analyte directly attached by the manufacturer. The purpose of the reference channel is to verify the function of binding on the sensor element surface via second receptor by augmenting the sample with magnetic particles with well defined binding properties. The reference channel may have an independent inlet or may be connected to the measuring channel.

In case of a sandwich assay, attachment of the magnetic particle by binding of the analyte (i.e. analyte-the first receptor-magentic particle-complex) to the sensor element surface generates the desired increase of signal. Hence, in order to create a maximum binding of particles on the sensor surface, the analyte already attached to the magnetic particles is stored in the device during production in the reference channel of the device. During operation, once the reference channel is flushed with the sample fluid, the magnetic particles get dissoluted and transported to the magnetic sensor element surfaces, where they bind to second receptor via the analyte. The more of these pre-prepared analyte magnetic particles bind, the higher the signal. Since all magnetic particles are carrying the analyte virtually every magnetic particle can bind. This should yield very high coverage of the sensor surface, subsequently high signals and thus allows to verify the function of the binding reaction by means of second receptor on the sensor surface.

In the case of a competitive assay, the function of the binding of free, unlabelled analyte to second receptor can be verified by the same means. In the competitive mode the decrease of signal previously caused by a defined amount of analyte labelled to magnetic particles and bound to second receptor is measured, which decrease is the result of replacing the labelled analyte, by unbound, competitive analyte upon the latter's addition to the device. In the ordinary competitive reaction, the number of magnetic particles bound to second receptor via analyte decreases, since the analyte competes for the same binding site ( second receptor) as the antibody/antigen. Hence, the decrease of the signal of the magnetic sensor signal is expected. The binding reaction whose function has to be verified is, however, still binding of the analyte to second receptor. In order to reproducibly generate a reasonable magnetic signal it is, hence, straightforward to again label the analyte with the magnetic particle and to measure the positive signal as for the sandwich assay in the reference channel or to measure signal decrease in a competitive mode in the reference channel.

In the first case, opposite to the ordinary behaviour of the competitive assay which may be performed in the "measuring" channel, the resulting high coverage of the sensor surface with subsequent increase of magnetic sensor signal is the desired measurement in the reference channel to verify function of the binding reaction by means of second receptor on the magnetic sensor surface. In the latter case, the competitive mode can be used to directly compare measurement channel and reference channel.

Reference channel (1.3) and measurement channel (see the channel on the right hand) are illustrated schematically in Figure 1.

In a thirteenth preferred embodiment of the device according to the first aspect of the present invention, the device further comprises a means (e.g. EEPROM, RFID, Bar Code or Data Matrix Code) for identification or data storage of information about the test to be run (e.g. identification of the device parameters, calibration curves).

For storage of device identification (including "traceability" information), manufacturing information (date, time, batch etc), test parameters (type of test, thresholds, calibration curves) electronic storage chips can be used. These are available e.g. in the Atmel EEPROM AT24Cxx family with small sizes ca. 5 x 3 x 0.9 mm³, storage capacities of 2 kB and two wire serial interface.

In a fourteenth preferred embodiment of the device according to the first aspect of the present invention, the device is designed for single use or is inexpensively fabricated, e.g. by mass fabrication processes such as injection moulding.

Since the detection process can easily be adapted to different tasks just by changing the coating of the magnetic particles and the sensor elements, the device can easily be configured for many different applications. In particular in POC diagnostics or personalized diagnostics cost and timing is an important issue. For that reason the device is designed in such away that it can be fabricated with mass production processes such as by micro-injection molding in a polymer material like PS, LCP and by automated joining and assembly. The device is in this embodiment a disposable device.

In a fifteenth preferred embodiment of the device according to the first aspect of the present invention, the device is miniaturized, for example with dimensions of 10-100, preferably 20-60 mm length, 5-50, preferably 10-25 mm, width and 1-10 mm, preferably 2-5 mm, height.

To make a small handheld instrument feasible, the device has to be as small as possible while retaining a size that can still be handled manually. Hence in the preferred embodiment the size of the device is about 60 (+- 10%) x 20(+- 10%) x 3(+- 10%) mm³.

This is illustrated in the preferred embodiment of Figure 13.

In a sixteenth preferred embodiment of the device according to the first aspect of the present invention, the device is made of polymer material, e.g. LCP, PS.

Polymer materials suitable for forming the device are well in the art. Preferred polymers are LCP (liquid crystal polymers), PS (polystyren), PC (polycarbonate), PP (polypropylene), COC (cyclic olefine copolymer), COP (cyclic olefine polymer).

In a second aspect, the present invention provides an instrument for detecting an analyte in relation to one or more bound magnetic particle labels, the instrument comprising the device of the present invention, and one or more means selected from
a) a means to mechanically, electrically and fluidically connect the instrument to the device,
b) a means to control fluidic flow in the device,
c) a means to control and/or record generation of variable magnetic field,
d) a means to concentrate a magnetic field in the vicinity of the magnetic sensor element, which means may be part of the instrument or the device,
e) a means to operate and read-out the magnetic sensor element,
f) means to read out the identification or data storage of the device according to claim 10 and applying this information to configure its setup or way of operation.
g) a means to evaluate and/or record magnetic sensor element signals,
h) a means to calculate and/or record analyte concentrations from these signals,
i) a means to present analysis results to a user,
j) a means to input user commands,
k) a means to transfer data by interfaces,
l) a means to be operated by battery or line connector,
m) a magnetic field generating element placed downstream from the magnetic sensor element(s), either inside or outside the device, the element suitable for washing by bidirectional pumping
n) an electromagnetic means that is temporarily operated over the sensor or a permanent magnet that is temporarily moved over the sensor, both useful for lifting unbound magnetic particles from the sensor surface(s), i.e. magnetic washing.

The device of the first aspect of the present invention can be inserted into an instrument for detecting an analyte in relation to bound magnetic particle labels.
The instrument may contain a slot into which the device is being inserted.
During insertion a spring contact array to mechanically and electrically connect the instrument to the device, will make contact with pads on the device, thus connecting the magnetic sensor element and the identification, storage chip to the instrument electronics. Moreover, the instrument may contact the magnetic sensor element on the device wirelessly.
On the other hand the fluidic channel structure of the device may be fluidically connected via a seal to the pump in the instrument.
An instrument software running on the microcontroller in the instrument may read out the identification in the identification chip and additionally test specific configuration data stored on the chip. These may encompass the traceability information, the type of the test, but also data that configure the instrument for performing the test (e.g. incubation time) or interpreting the data (e.g. slope and offset of a calibration curve).
The instrument electronics preferably comprises of a low power micro controller with integrated analog/digital converters, digital 10 ports and bus driver (e.g. USB, I²C) analogue electronics for sensor supply and control (e.g. Wheatstone bridges), amplifiers, filters, DC/DC converters etc.. The magnetic field generator may be realized as coil with core tapering off to flux concentrators above the sensor. The user interface may consist of a set of keys and an alphanumeric (e.g. seven segment) or graphical (e.g. LCD, OLED) display.
The instrument may be operated with the integrated rechargeable battery or when stationary attached to an external power source.

In a first preferred embodiment of the instrument according to the second aspect of the present invention, the instrument additionally comprises a means to store analysis data, e.g. a flash memory.

In this embodiment, the analysis data are being stored in a local memory in the instrument such as a flash memory or an EEPROM.

In a second preferred embodiment of the instrument according to the second aspect of the present invention, the instrument additionally comprises a means to read out the identification or data storage of the device if present and the instrument applies this information to configure its setup or way of operation.

The microcontroller of the instrument in this embodiment reads out the identification/storage chip on the device and uses this information to configure the instrument for the measurement. In the most simple case this means identifying type of test and an identification number. But it may also imply more sophisticated configuration steps such as deriving thresholds, calibration curve parameters (e.g. slope, offset) and the like from these data.

In a third preferred embodiment of the instrument according to the second aspect of the present invention, the instrument comprises a means to generate a magnetic field in the vicinity of the channel structure of the device.

In a third aspect, the present invention provides a method for quantitative and/or qualitative measurement of an analyte bound to magnetic particles in a sample comprising introducing a sample into the device of the present invention, optionally inserting said device into the instrument of the present invention and detecting the magnetic particles bound to the magnetic sensor, e.g. by generation of a variable magnetic field and measuring of the distortion of the magnetic field generated by the specifically bound particles by means of the magnetic sensor element(s).

In an illustrative embodiment of the third aspect of the present invention, after insertion of the sample the device is inserted into the instrument. The instrument reads out the identification/memory chip, to identify the type of test implemented on the device and use the data to configure the instrument for the test. Meanwhile the sample flows into the channel structure on the device through a means for pre-processing such as a filter or a means for mixing an anticoagulans with the sample until it reaches a chamber where precoated magnetic particles are stored. These get dissolved, subsequently mix with the sample and the analyte binds to the magnetic particles carrying a suitable receptor (the first receptor). The reaction product (analyte bound to magnetic particle with the first receptor) gets further transported in the flow and finally guided by suitable means in the channel structure onto the sensor element. There magnetic particles carrying the analyte bind to sensor element surfaces carrying second receptor. Unbound particles get removed by alternately pumping the sample volume over the sensor element back and forth between sensor element and magnetic field generator over the channel structure where they get held by magnetic forces. During flow of the sample through the channel structure the instrument control initiates generation of a variable magnetic field over the sensor. At the beginning this is used to test sensor function and detect the flow of particles across the magnetic sensor element. After the particles have bound specifically to the sensor element surfaces a sensor signal of different quantity or quality can be measured from which the number of bound particles can be derived. This signal is being processed and the results are presented to the user. Finally the device is removed from the instrument.

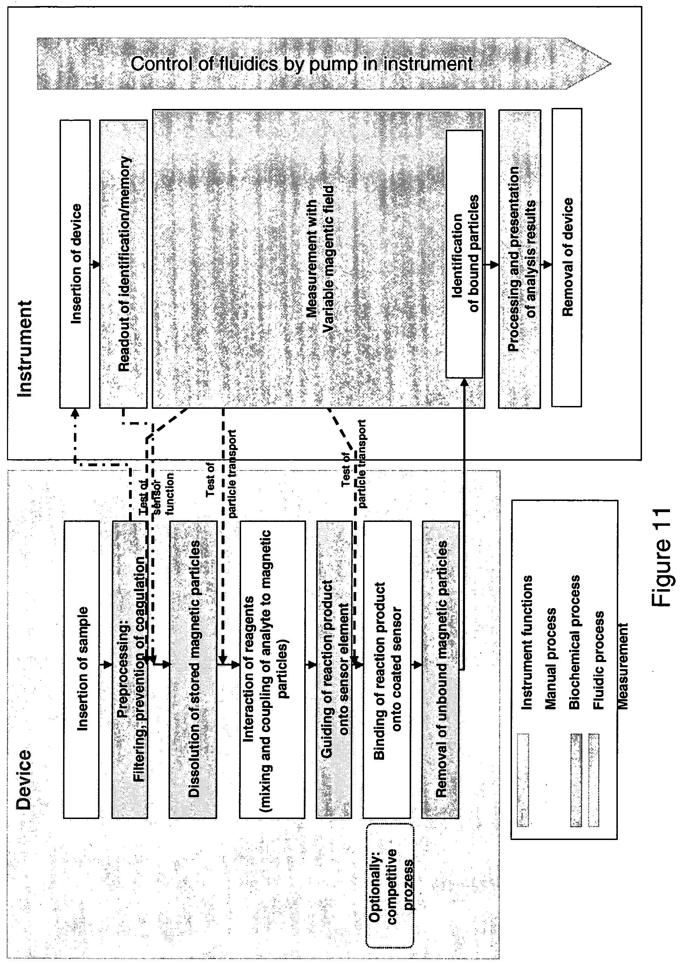

Figure 11 illustrates the process flow of this embodiment.

In a first preferred embodiment of the third aspect of the present invention, the method further comprises additionally calculating the number of the bound particles from the resulting sensor signal by the controller in the instrument.

With a high-resolution sensor, whose layout has been tailored to the size and magnetic properties of the magnetic particles single particle detection becomes feasible. The magnetization curve of the magnetic particle can be identified by means of magnetization measurements. The magnetic sensor element characteristic is tailored to this curve by optimization of the GMR stack and layout. Hence high sensitivities can be achieved that allow to precisely derive the number of bound particles down to a single particle from the sensor signal. This has been demonstrated by Prof. Hüttens group at Bielefeld University.

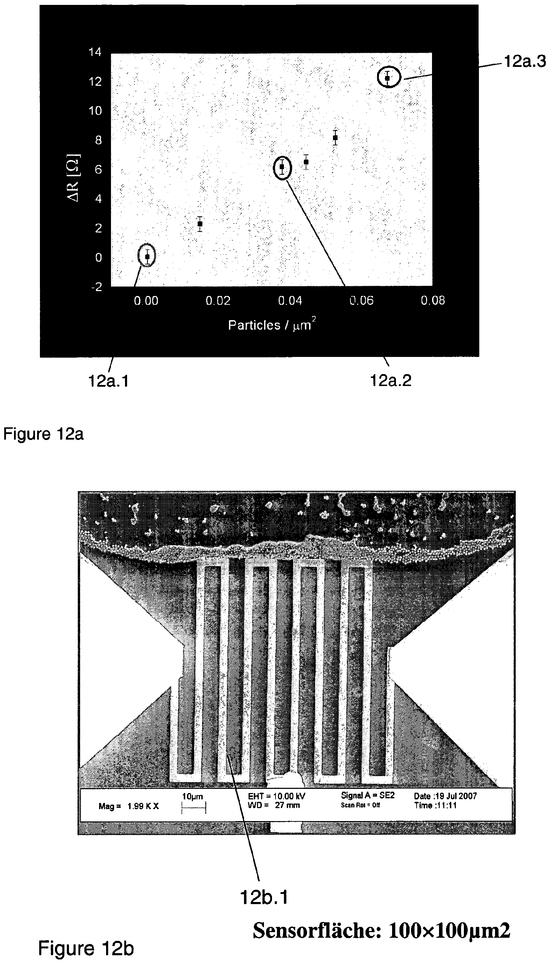

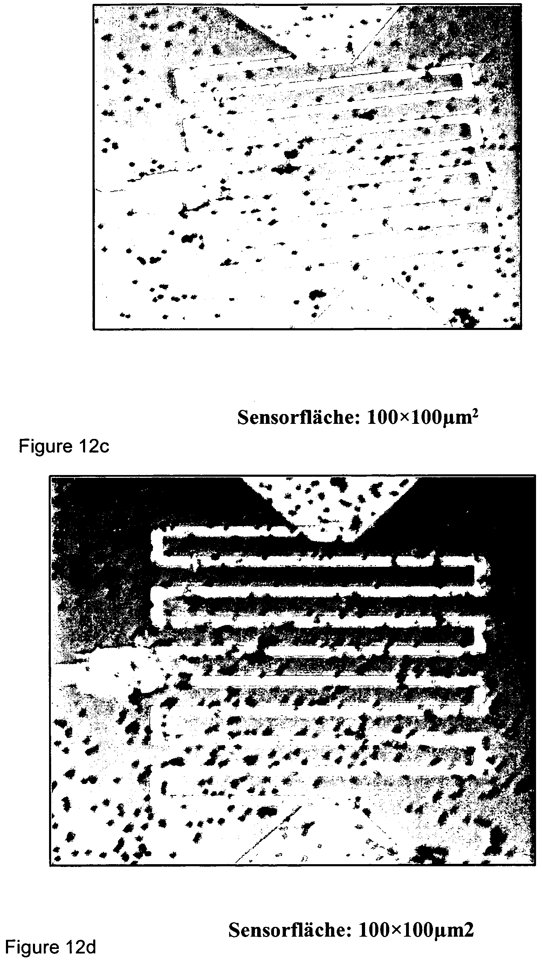

Figure 12 shows measurement results with a meander shaped GMR sensor with 100 µm x 100 µm area and 1 µm magnetic particles at different particle concentrations. The x-axis is the number of particles per µm², the y-axis is the change of resistance of the GMR-sensor. The lowest particle concentration is illustrated in figure 12b with a single particle on the sensor (12b.1), leading to low but distinctive change of resistance (12a.1). Figures 12.c and 12.d show higher particles concentrations on the sensor, that lead to higher changes of resistance (12a.2 and 12a.3).

In a second preferred embodiment of the method of third aspect of the present invention, the method further comprises additionally presenting the measurement results on the display of the instrument.

In this embodiment, the measurement result is presented on the display of the instrument, e.g. as message "analyte is below/exceeds threshold", numerically as value or graphically as concentration curve.

In a third preferred embodiment of the method of the third aspect of the present invention, the method is one, wherein removal of unspecifically bound magnetic particles on the magnetic sensor element surface is achieved by
a) unidirectional pumping, i.e. pumping sample fluid without magnetic particles across the sensor, thereby lifting unbound magnetic particles from the sensor surface.
b) bidirectional pumping, i.e. repeatedly pumping the sample from the magnetic sensor surface to a magnetic field generating element placed downstream from the magnetic sensor element(s) and back, thereby lifting the unspecifically bound particles from the sensor surface and transporting them above the magnetic field generating element where they are retained, while the sample fluid is moved back to the sensor surface with the return stroke of the pump or
c) magnetic washing, i.e. generating a magnetic field above the magnetic sensor surface that lifts unbound magnetic particles from the sensor surface. This can be achieved by an electromagnetic means that is temporarily operated over the sensor or by a permanent magnet that is temporarily moved over the sensor.

Figure 10 shows a segment of the channel structure (10.1) with the magnetic sensor element (10.2) with bound magnetic particles (10.3) and unbound magnetic particles (10.4). An alternating flow (10.1) removes the unbound magnetic particles and transports them to the magnetic field generator (10.5) where they are held (10.6) by magnetic forces (bidirectional pumping).

In embodiments with unidirectional pumping, sample fluid not laden with magnetic particles is used to flush unbound particles from the sensor surface. The sample fluid arriving at the sensor element depletes from magnetic particles after all magnetic particles in the magnetic particle reservoir have been dissolved. The point in time when this occurs can be measured with the magnetic sensor element and the pump can be controlled accordingly.

In a fourth preferred embodiment of the method of third aspect of the present invention, the method is one, wherein fluidic flow in the channel structure and the function of the magnetic sensor element are verified by measuring the concentration profile of the magnetic particles in the sample flow over the length of the channel structure.

If the magnetic particles are stored with saccharose to allow a homogenous particle distribution in the sample in the channel structure, they get dissolved as the sample flow runs through the columns. This will lead to a magnetic particles concentration profile over the length of the channel structure. As the sample flow front reaches the magnetic sensor element this will detect this increase of concentration of magnetic particles due to its high sensitivity. By this means by measuring this concentration profile both the flow and the function of the magnetic sensor element can be verified.

In a fifth preferred embodiment of the method of third aspect of the present invention, the method is one, whereby the functional or quantitative binding of the second receptor or analyte on the magnetic sensor surface is being verified by providing a device with stored magnetic particles specifically coated according to claim 9, wherein the sample flow is also being branched into the reference channel thereby leading the coated particles over the specifically coated magnetic sensor in the reference channel, whose signal indicates if specific binding has occurred, thus yielding the indication that the functional or quantitative binding of the second receptor is working.

As shelf life of such a device is an important issue, it is important to detect potential degradation of reagents. For that reason the device can be equipped with an internal control reagent or with a parallel channel structure. Behind the inlet and sample preprocessing unit the fluidic channel branches into two channels, the already describes analysis channel and the so called reference channel. The reference channel is identical to the analysis channel, except the coating of the magnetic particles stored in its respective chamber. These particles are directly coated with the analyte. As they get dissolved and transported over the sensor, they will directly bind to the sensor if the reagents are not degraded. As before this binding can be detected by measuring the signal created by the bound magnetic particles. If this is above a certain threshold it is safe to assume that the biochemistry in the device is fully operational.

In a sixth preferred embodiment of the method of third aspect of the present invention, the method is one, wherein the read-out of the identification or data storage on the device by the instrument according to claim 13 is being used for identification of the test being run or for configuration of the instruments settings or operation.

In a fourth aspect, the present invention provides a method for fabricating a device of the present invention comprising
a) providing at least two injection moulded or embossed polymer plates,
b) either directly generating a magnetic sensor element in at least one channel wall or integrating a discrete magnetic sensor element in at least one channel wall,
c) introducing electrical conductors or equivalents thereof to contact said magnetic sensor element
d) joining the at least two injection moulded or embossed polymer plates to form a fluidic channel structure in the device.

In a first preferred embodiment, the method for fabricating a device according to the fourth aspect may comprise
a) providing an injection moulded polymer base plate comprising an orifice for insertion of the magnetic sensor element and a two component injection moulded top plate comprising the fluidic channel structure in the electrically insulating polymer and electrical conductors for contacting the magnetic sensor element component in an electrically conducting polymer preferably coated with a metallic conducting material; in this embodiment the step c) of introducing electrical conductors to contact said magnetic sensor element takes place upon formation of the top plate,
b) integrating a discrete magnetic sensor element in the base plate,
d) joining the two injection moulded polymer plates to form a fluidic channel structure in the device; in this embodiment the shape of the channel structure is formed by the top plate with the bottom surface being provided by the base plate.

The base plate (13.1) of the device may be fabricated in single shot injection moulding containing as structures grooves (13.2) cicumscribing the fluidic structure contained in the top plate, orifices (13.3, 13.4) for the sensor element (13.5) and the identification/memory (13.6) chip, Clamping elements for mechanically clamping top and base plate. The top plate (13.7) is fabricated in two shot moulding from a conductive and non conductive polymer (preferably LCP). In the noncondutice part the, the inlet (13.8), the sample preprocessing (13.9), channel structure and conterparts for the mechanical clamping elements are formed. The conductive polymer forms the condiuctors to which the sensor and identification/memory chip are connected and which are routed to the outside to form contact pads (13.10) for contacting with the instruments. These conductors are coated in separate electroforming process with a metal (e.g. Au).

Figure 13 shows an embodiment of the device, with the base part (13.1) containing a groove for sealing (13.2) around the channel structure, orifices for identification chip (13.3) and the magnetic sensor element (13.4). The top part (13.7) contains the orifice (13.8) with filter element (13.9) for sample insertion and the electrical contacts (13.10) for connecting the magnetic sensor element (13.5) and the memory chip (13.6) to the instrument. The channel structure is sealed with a trace of adhesive (13.11) between top part and base part.

In a second preferred embodiment, the method for fabricating a device according the fourth aspect may comprise
a) providing an injection moulded polymer base plate comprising an orifice for insertion of the magnetic sensor element and an injection moulded top plate comprising the fluidic channel structure and electrical conductors generated on its surface preferably by selective electrodeposition, thin film technology or by printing conductive ink or adhesive for contacting the magnetic sensor element; in this embodiment the step c) of introducing electrical conductors to contact said magnetic sensor element takes place upon formation of the top plate,
b) integrating a discrete magnetic sensor element in the base plate,
d) joining the two injection moulded polymer plates to form a fluidic channel structure in the device; in this embodiment the shape of the channel structure is formed by the top plate with the bottom surface being provided by the base plate.

Alternatively to the two shot moulded top plate of the first preferred embodiment, the top plate can also be realised as a single shot moulded non conducting polymer part onto which conductive layers are deposited with selective electrodeposition, thin-film technology or printing of conductive ink or adhesive.

In a third preferred embodiment, the method for fabricating a device according to the fourth aspect may comprise
a) providing an injection moulded polymer base plate comprising an orifice for insertion of the magnetic sensor element and an injection moulded top plate comprising the fluidic channel structure,
b) integrating a discrete magnetic sensor element in the base plate,
c) introducing electrical conductors to contact said magnetic sensor element, wherein the electrical conductors are on a flexible printed circuit board,
d) joining the two injection moulded polymer plates and the flexible printed circuit board to form a fluidic channel structure in the device.

Another alternative to the first preferred embodiment as set forth in this third preferred embodiment would be to put the conductors on a separate substrate e.g. a thin flexible printed circuit board that would be positioned between the top and bottom part and lead outside to yield the external contacts to the instrument.

The method for fabricating a device of the present invention may comprise directly generating the magnetic sensor element on the polymer material in the channel structure of the device. This generating step may replace the step of integrating a discrete magnetic sensor element in one or more of the embodiments of the fourth aspect of the invention.

Directly generating the magnetic sensor element on the polymer material in the channel structure of the device may be performed by either thin-film deposition or printing of sensing polymer materials, preferably polymer electronic materials or polymer nanocomposites.

Direct generation of GMR sensors on polymer material with thin-film technology has been described in ("Magnetostrictive GMR sensor on flexible polyimide substrates", T. Uhrmann, b, L. Bär, T. Dimopoulos, N. Wiese, c, M. Rührig and A. Lechner,Journal of Magnetism and Magnetic Materials Volume 307, Issue 2, December 2006, Pages 209-211). This process can be favourably combined with the above described flexible printed circuit board (base material also polyimide) as conductive layer or be extended to directly process the GMR layers on the any of the polymer parts of the device that form part of the channel structure.
Moreover, magnetic sensor elements made of GMR layers can be directly generated on any of the polymer parts of the device that form part of the channel structure by depositing polymer nanocomposites. (Z. Guo, S. Park, H. T. Hahn "Giant magnetoresistance behavior of an iron/carbonized polyurethane nanocomposite" Appl. Phys. Lett. 90, 053111 (2007)).

The magnetic sensor element in the present invention may be combined with an equivalent of electrical conductors such as a circuitry for wireless transmission of electrical power or sensor signals similar to RFID chips (Turgut S. Aytur, Tomohiro Ishikawa, and Bernhard E. Boser, "A 2.2-mm2 CMOS Bioassay Chip and Wireless Interface", 2004 Symposium on VLSI Circuits, Digest of Technical Papers, pages 314-317).

The method for fabricating a device of the present invention may additionally comprise
e) sealing of the channel structure.

Sealing of the channel structure may be performed by dispensing adhesive along the channel structure, along the part of the magnetic sensor element serving as wall of the channel structure and along chambers.

In figure 13, top and bottom plate are sealed by dispensing adhesive (13.12) into the groove running around the channel structure and across the sensor to separate the sample flowing over the sensor from the conductors.

The method for fabricating a device of the present invention may comprise the step
f) coating the sensor element surface with second receptor.

Coating the sensor element surface with second receptor may be performed by dispensing, pin transfer or contactless dispensing (e.g. jetting as in a dispensing well plate).

This step f) may be employed before or after integrating a discrete magnetic sensor element in at least one channel wall or after directly generating a magnetic sensor element in at least one channel wall.

Preferably, the sensor element comprises several sensor element surfaces. These individual (distinct) sensor element surfaces may be coated with individual receptors 2 (i.e. second receptor, second receptor', second receptor", etc) in order to run several tests in parallel. In this embodiment on each sensor element surface a different analyte can be bound. The analytes are then labeled with "individual receptors 1" (i.e. the first receptor, the first receptor', the first receptor", etc).

Standard dispensing techniques as used in micro array production can be applied for specifically coating the sensor element surface. These devices achieve the required positional resolution by numerically controlled handling systems (e.g. SciFlexarrayer by Scienion AG). Another solution are monolithic dispensing heads where the nozzle arrangement is tailored to the sensor array geometry (e.g. Top Spot heads by BioFluidix GmbH).

The method for fabricating a device of the present invention may comprise the step of
g) dispensing a particle solution in respective position in the channel structure (walls or separate chamber) and drying said solution in that place.

In this embodiment, the specifically coated magnetic particles have to be integrated inside the device (according claim 2 and 3) within the flow path. The sample dissolves the magnetic particles and transports them to the sensor.

The shell of used magnetic particles can be made of e.g. Silica, PS, PMMA, PVA, or others with or without functional groups e.g. like sulfur-, amino-, carboxyl-, sulfate- or epoxid- functions. The shell is coated with the specific reagents by e.g.
- direct (adsorptive) coating or
- chemical linking or
- on a modular basis like pre coating with e.g. Streptavidin/Biotin or anti-IgG (also adsorptive or chemical)
- For the manufacturing of the magnetic particles a specific reactor with magnetic separation tools or continuous membrane flow concentration units can be used.

To ensure the resolvability and stability of the magnetic particles reagents e.g. like sucrose, glycol or glycerin and proteins e.g. like BSA, IgG or Casein and preservatives e.g. like sodium acid, N- methyl-isothiazolone and or 2-chloracetamide are used.

For the dispensing process the magnetic particle solution has to be homogeneous (stirred) and the dispensed volume must be accurate to ensure reproducibility of test results. The magnetic particle volume can be in a range of 0.02-5 µl ±4%. The amount of magnetic particles is assay specific. The dispenser has to ensure the correct positioning (in a range of ±10µm). After dispensing of the magnetic particles a drying process controlled by temperature and humidity can be applied to ensure stable reagents in the device.

Figure 3 shows a coated magnetic particle consisting of magnetic core particles (3.1) surrounded by a shell (3.2), whose surface is activated (3.3). A linker molecule (3.4) connects the first receptor (3.5) to the magnetic particle.

In a fifth aspect, the present invention provides the use of the device according to the present invention or of the method of detecting according to the present invention or of the instrument of the present invention for the detection and/or measurement of organic or inorganic analytes. Preferably these analytes are analytes for which a receptor assay can be designed.

Preferred analytes are
▪ proteins, enzymes, DNA, oligonucleotides, antibodies, allergens
▪ Microorganisms [e.g. eukaryotic, prokaryotic cells; e.g. bacterial, fungi, algae, protozoa, virus, phages]
▪ Cells (e.g. out of blood or tissue) and cell parts.
▪ Free or bound haptenes (e.g. steroids, hormons drugs, lipids saccharid, pesticides, fungicide pharmaceuticals)
▪ Materials which can generate antibodies as well as other chemical substances.

To detect such analytes the use of binding proteins e.g. antibodies, receptors, DNA, phage proteins and others are useful. The reagents to be used are already described in technologies like ELISA, RIA, ELIA, receptor tests an others, Especially modular systems (Streptavidin/Biotin, Ni-NTA/HisTag or IgG-based) have advantages with regard to the magnetic particle or sensor surface coating. With such modular systems universal magnetic particles and sensor surfaces can be provided.
The same test concepts like competition or sandwich assays and their variations are usable for the present invention.

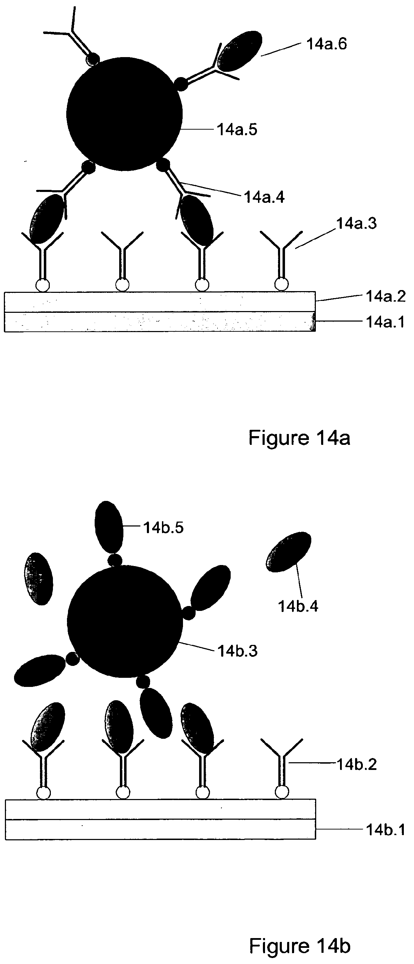

Figure 14a shows a sandwich assay where the first receptor (14a.3) is bound on a strepavidine layer (14a.2) coated on a gold layer (14a.1). The magnetic particle (14a.5) is attached via the analyte (14a.6) that is bound to second receptor (14a.4).
Figure 14b shows a competitive assay. second receptor (14b.2) is coated onto a gold surface with streptavidine (14b.1). As analyte (14b.4) and antigene/antibody (14b.5) attached to the magnetic particle (14b.3) compete for the binding sites on second receptor (14b.2) the more analyte is present, the less magnetic particles can bind.

The use of the present invention is preferably in diagnostic fields of one of
▪ Food (ingredients/bacterial)
▪ Human (personalized diagnostics, point of care testing or emergency testing)
▪ Veterinary
▪ Ecological
▪ Hazardous
▪ Pest management
▪ Drug of abuse diagnostic
▪ Aerospace or Prevention of terrorist attacks.

The use of different solid materials (magnetic particles, micro plates, tubes etc.) to bind immunological complexes and to detect light or fluorescence to measure a specific analyte are well known and are described in a lot of patents and papers.
Most of these systems are designed for use in clinical labs or lab organizations for medium or high sample throughput. A few systems exist as table top for low sample throughput (single sample measurement) based on the same technology. Dependent on the detection technologies the systems are expensive and educated personal is needed to run the systems and they are usable only stationary.
Other technologies as lateral flow (test strips) are available. The mobile use is the big advantage but most of them give only qualitative results and not all tests are applicable. For all the here shown areas the following major features are required.
▪ Mobile systems
▪ simple to use (no educated personal needed)
▪ time to result below 15 min
▪ qualitative and quantitative results
▪ low costs for the instrument, device and assays
▪ no service necessary
▪ panel testing if possible (2-8 parameters with one device)

The here used technology can provide all these benefits dependent on the detection method and the specific device in combination with a small mobile instrument.

### Figures of the present invention

Figure 1 schematically shows a preferred embodiment of the device of the present invention. The orifice (1.1) for insertion of the sample leads via an analysis channel structure (1.2), a chamber (1.4) for storage of magnetic particles and a means for mixing (1.5) to the magnetic sensor element (1.6). Behind the magnetic sensor element follows a magnetic field generation element (1.7) and a waste chamber (1.8). A reference channel structure (1.3) of identical layout runs parallel to the analysis channel structure (1.2). Conductors (1.9) allow to connect the magnetic sensor elements (1.6) and the identification/memory element (1.10) to the instrument.
Figure 2a shows a segment (2a.1) of the channel structure with a preferred embodiment of the means for dissolution of the magnetic particles. It consists of a chamber (2a.2) with columns (2a.3) on which the magnetic particles are coated.
Figure 2.b shows results of a simulation in the plane of chamber 2a.2 indicating that particles coated on columns 2a.3 are dissolved by the fluid and evenly distributed. The arrows represent flow direction, while the colours represent magnetic particle concentration with dark grey being lowest and light grey being highest concentration.
Figure 2c shows results of a simulation of magnetic particle concentration in one half of the channel cross section. The units of the x- and y-coordinates are 0.1 mm; the colour indicates magnetic particle concentration, with dark grey being lowest and light grey being highest concentration.
Figure 3 shows a coated magnetic particle consisting of magnetic core particles (3.1) surrounded by a shell (3.2), whose surface is activated (3.3). A linker molecule (3.4) connects the first receptor (3.5) to the magnetic particle.
Figure 4a shows a segment of the channel structure with reduction of the channel height (4a.1) above the sensor element (4a.2), to accelerate sedimentation and thus binding of the magnetic particles (4a.4) on the sensor element surfaces (4a.3).
Figure 4b shows a curved segment (4b.3) of the channel structure (4.b.5), where magnetic particles (4b.4) get deflected due to inertia and land on the sensor element surfaces (4b.2) of the sensor element (4b.1).

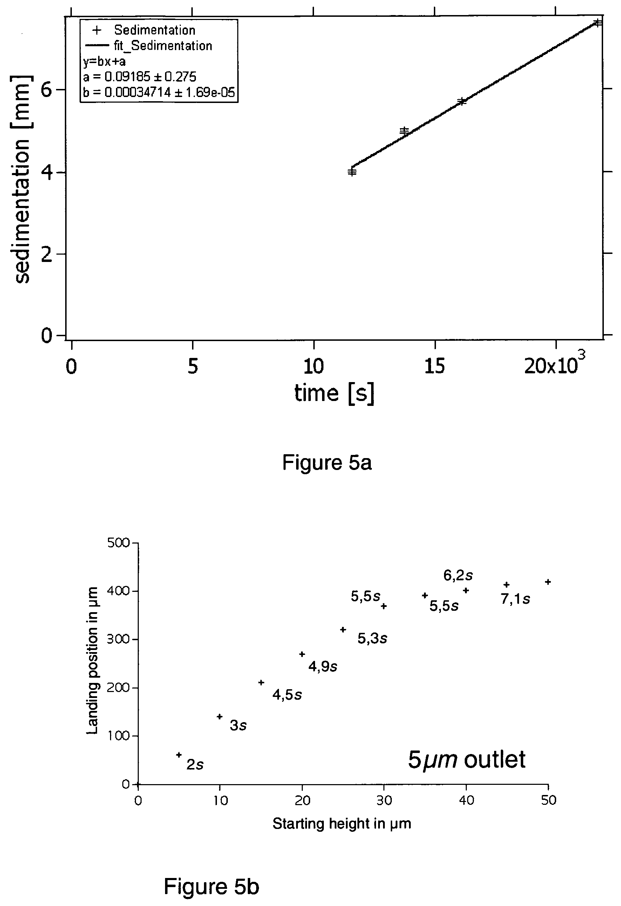

Figure 5a shows results of sedimentation experiments with 1 µm sized magnetic particles. The x-axis units is sedimentation time in 10³ s, the y-axis shows the starting height of the sedimentation experiment in Millimetres.
Figure 5b shows simulation results of the channel structure with height reduction over the magnetic sensor element. The inlet height of the channel was 50 µm, its outlet height 5 µm. Different starting heights of the magnetic particles (x-axis) were assumed. The y-axis denotes the landing position measured from the entry of the height reduced channel part. The numbers at the data points indicate the sedimentation time in seconds.
Figure 6 shows the channel segment with the height reduction over the magnetic sensor element. The channel inlet (6.1) is reduced in height (6.2) above the magnetic sensor element (6.3) and widened to the outlet (6.4).
Figure 7a shows fluid dynamics simulations at different scales. In the lower image at a channel of 10 mm turbulent flow can be observed and thus intensive mixing be expected. In the upper image with channel width reduced to 1 mm all other variables remaining unchanged only laminar flow can be observed and thus poor mixing only due to Brownian motion can be expected.
Figure 7b shows a segment of the channel structure (7b.1), where columns of different height (7b.2) are distributed in order to improve laminar mixing by separating, folding and reuniting fluid flow.
Figure 7c shows a segment of the channel structure with a chamber (7c.1) with a movable member (7c.2).
Figure 8 schematically shows a cross section of the device where ferromagnetic elements (8.3) are integrated to the left and to the right of the channel structure (8.1) in order to achieve magnetic field concentration over the magnetic sensor element (8.2).
Figure 9 schematically shows two variants of the coating of a sensor element surface. In one case a) an activated second receptor (e.g. sulphur activated) is directly bound on a gold layer (9.1). Another option is a surface activation (9.2) e.g, streptavidine on which second receptor (9.3) is bound with the biotin streptavidine bond.
Figure 10 shows a segment of the channel structure (10.1) with the magnetic sensor element (10.2) with bound magnetic particles (10.3) and unbound magnetic particles (10.4). An alternating flow (10.1) removes the unbound magnetic particles and transports them to the magnetic field generator (10.5) where they are held (10.6) by magnetic forces.
Figure 11 illustrates the process flow. After insertion of the sample the device is inserted into the instrument. The instrument reads out the identification/memory chip, to identify the type of test implemented on the device and uses the data to configure the instrument for the test. Meanwhile the sample flows into the channel structure on the device through a means for pre-processing such as a filter or a means for mixing an anticoagulans with the sample until it reaches a chamber where precoated magnetic particles are stored. These get dissolved, subsequently mix with the sample and the analyte binds to the magnetic particles carrying a suitable receptor (the first receptor). The reaction product (analyte bound to magnetic particle with the first receptor) gets further transported in the flow and finally guided by suitable means in the channel structure onto the sensor element. There magnetic particles carrying the analyte bind to sensor element surfaces carrying second receptor. Unbound particles get removed either unidirectionally or bidirectionally pumping sample volume across the sensor. Bidirectional pumping means alternately pumping the sample volume over the sensor element back and forth between sensor element and magnetic field generator over the channel structure where particles in the fluid get held by magnetic forces. During flow of the sample through the channel structure the instrument control initiates generation of a variable magnetic field over the sensor. At the beginning this used to test sensor function and detect the flow of particles across the magnetic sensor element. After the particles have bound specifically to the sensor element surfaces a sensor signal of different quantity or quality can be measured from which the number of bound particles can be derived. This signal is being processed and the results are presented to the user. Finally the device is removed from the instrument.
Figure 12a shows measurement results with a meander shaped GMR sensor with 100 µm x 100 µm area and 1 µm magnetic particles at different particle concentrations. The x-axis is the number of particles per µm², the y-axis is the change of resistance of the GMR-sensor.
   The lowest particle concentration is illustrated in figure 12b with a single particle on the sensor (12b.1), leading to low but distinctive change of resistance (12a.1).
Figures 12.c and 12.d show higher particles concentrations on the sensor, that lead to higher changes of resistance (12a.2 and 12a.3).
Figure 13 shows an embodiment of the device, with the base part (13.1) containing a groove for sealing (13.2) around the channel structure, orifices for identification chip (13.3) and the magnetic sensor element (13.4). The top part (13.7) contains the orifice (13.8) with filter element (13.9) for sample insertion and the electrical contacts (13.10) for connecting the magnetic sensor element (13.5) and the memory chip (13.6) to the instrument. The channel structure is sealed with a trace of adhesive (13.11) between top part and base part.
Figure 14a shows a sandwich assay where the first receptor (14a.3) is bound on a strepavidine layer (14a.2) coated on a gold layer (14a.1). The magnetic particle (14a.5) is attached via the analyte (14a.6) that is bound to second receptor (14a.4).
Figure 14b shows a competitive assay. second receptor (14b.2) is coated onto a gold surface with streptavidine (14b.1). As analyte (14b.4) and antigene/antibody (14b.5) attached to the magnetic particle (14b.3) compete for the binding sites on second receptor (14b.2) the more analyte is present, the less magnetic particles can bind.

### Examples

### Example 1: Simulation of the dissolution of magnetic particles in the reservoir

Objective: Assessment of dissolution behaviour of magnetic particles stored in a reservoir.

Method: The magnetic particle reservoir (see figure 2) was modelled as cylindrical cavity of 4 mm diameter, with a 300 µm wide inlet and outlet channel. The overall height of this fluidic structure is 300 µm. Attached to the ceiling of this chamber are cylindrical columns of 200 µm width and 200 µm height, whose lateral and top surfaces are coated with magnetic particles. This arrangement was simulated with an input flow velocity of 100 µm/s. The simulation tool was Comsol Multiphysics by Femlab.

Results: Figure 2b and 2c show simulation results, with the colours indicating normalised concentrations and arrows flow directions. Light colours indicate high concentration, dark colours respectively low concentration. Figure 2b shows a planar view of half of the reservoir that indicates that the coated particles on the lateral surfaces of the columns get nicely dissolved and lead to relatively uniform particle distribution in the plane. (Remark: The negative concentration values in figure 2b are due to numerical problems during the simulation.) Figure 2c shows the simulated vertical concentration in the left half of the outlet channel. It illustrates a good distribution across the channel cross section with a first maximum close to the middle and a second maximum at the channel floor. This second maximum results from the particles coated to the top surfaces of the columns. These sediment quicker than the ones coated to the lateral column surfaces.

Overall, these simulation results indicate that a magnetic particle reservoir of this design and coated this way is a viable means to achieve a good dissolution of particles across a channel cross section.

### Example 2: Assessment and optimisation of particle sedimentation

Objective: Assessment and optimisation of sedimentation of magnetic particles on the sensor surface.

Method: At first sedimentation speed of 1 µm magnetic particles in a standing solution was determined experimentally. As the measured sedimentation speed proved insufficient for the application in the disposable the channel geometry was altered to accelerate sedimentation on the sensor surface. The inlet channel with 50 µm height and 80 µm width was gradually reduced over a length of 400 µm to 10 µm height and 400 µm width at the outlet channel by means of a kind of a linear inverse ramp (see figure 6). Sedimentation time of an aqueous solution with 1 µm magnetic particles uniformly distributed over the cross section streaming laminarly with a velocity of 100 µm/s at the inlet was calculated. The simulation tool was Comsol Multiphysics by Femlab.

Results: Figure 5a shows the results of the sedimentation experiments. The x-axis depicts the sedimentation time in 10³ seconds, the y-axis the starting height of the sedimentation experiment. Extrapolation of these results to lower heights indicates that in a standing medium due to gravity alone even at heights of a few hundred micron sedimentation still takes several minutes. This is too long for a disposable that should run entire tests in only a few minutes. Figure 5b shows the results of the simulation of particle sedimentation during flow through the above outlined inverted ramp. The x-axis indicates the starting height of the particle; the y-axis the landing position measured from the beginning of the inverted ramp, i.e. the end of the 50 µm by 80 µm channel. The numbers at the data points are the sedimentation time in seconds.

These results indicate that the inverted ramp is a perfect means to
1) drastically reduce sedimentation time to an acceptable level of a few seconds
2) achieve a uniform particle distribution on a clear cut surface.

### Example 3: Sensitivity tests with a GMR sensor

Objective: Assessment of GMR-sensor sensitivity for low bead densities on the sensor surface.

Method: A GMR-sensor by the Hütten group of Bielefeld university with 100 µm x 100 µm sensor surface and a sensor characteristic tailored to the magnetisation curve of 1 µm magnetic particles was characterised with different particle loads on its surface. To that end droplets with different particle concentrations were dispensed onto the sensor surface and dried. The sedimented particles on the sensor surface were counted under an optical microscope and the resulting GMR sensor signal under an external magnetic field was measured. In order to achieve very low particle sedimentation on the sensor besides the concentration the dispensing position of the drop on the sensor surface was varied. This way it could observed that during shrinkage of the droplet during the drying process, the droplet boundary was moving over the sensor surface dragging most of the particles with it and leaving only small numbers of particles on the sensor surface.

Results: Figure 12 c and d show the sensor surface with different particle loads. Figure 12d shows a microscope image of the experiment with a starting concentration of 0.2 mg/ml where the sensor was fully covered with droplet. It can be observed that (except some agglomerations) an almost uniform coverage of the sensor surface is achieved. The same situation, however, with a lower surface coverage can be observed in figure 12c. In this experiment the droplet was also put centrally on the sensor the concentration, however, was reduced to 0.1 mg/ml.

This concentration was kept invariant in the experiment whose result is illustrated in the microscope image of figure 12b. The droplet with the particle solution, however, was positioned in such a way that the sensor was on its rim. So when the droplet dried its boundary retracted over the sensor. The boundary of the dried droplet with its agglomeration of particles can still be seen in the top part of figure 12b. In the lower part of the image a single particle (12b.1) can be identified on the sensor. Its resulting signal could be measured with this GMR sensor.

From these measurements the curve in figure 12a shows the nearly linear relation between particle concentrations on the sensor surface (x-axis) in particles/µm² and resulting change of GMR-sensor resistance in Ω. The designated data points 12a.1 to 12a.3 correspond to the particle loads shown in figures 12b.1 to 12b.3 respectively.

These measurements indicate that single particle sensitivity is feasible with highly sensitive sensors such as GMR sensors specifically tailored to the magnetisation curve of the magnetic particles.

## Claims

1. Device for detecting an analyte directly or indirectly bound to one or more magnetic particle labels **characterized by**
A) at least one fluidic channel structure for transporting and processing magnetic particles, and
B) an integrated magnetic sensor element,
wherein
the magnetic sensor element is part of at least one channel wall,
the fluid channel structure geometry is optimized for fast and uniform binding of magnetic particle/analyte complexes on the sensor element surface, by leading a fluid flow over the sensor element surface, wherein the magnetic particle/analyte complexes are guided onto the surface of the magnetic sensor element
by
E1) an alteration of the fluidic flow profile such as by a gradual reduction of channel height (fig 4a.1) over the sensor or
E2) by inertial forces generated by guiding the flow in a curved channel (fig 4b.3) wherein the sensor element is positioned in a manner that it can be targeted by the flow, e.g. in the outer channel wall in the curve
and the magnetic sensor element is a GMR, TMR, CMR, EMR or Hall-sensor.

2. The device according to claim 1, further **characterized by**
C) a means for optimizing dissolution of magnetic particles, wherein the magnetic particles, which may or may not be coated with a first receptor and/or analyte, are released from the means for optimizing dissolution of the magnetic particles.

3. The device according to claim 1 or 2 further **characterized by**
D) magnetic particles coated with a first receptor which particles are stored in the device and are capable of binding to the analyte once a flow of the sample through the channel structure occurs.

4. The device according to any of claims 1-3, further **characterized by**
F) an element for mixing sample and magnetic particles to facilitate binding or competition of analyte to particle by means of a receptor, wherein the element for mixing is
F1) a passive structure such as a change of channel cross section or channel direction or a combination of both; or
F2) an active structure such as a ferromagnetic element lying loosely in the channel structure or in a chamber that may be moved by an alternating magnetic field.

5. The device according to any of claims 1-4, wherein the magnetic sensor surface is coated with gold to allow the specific binding of a second receptor and surrounding surfaces, are not gold second receptor, but coated with a different material, which is inert for binding to the first receptor, the second receptor or analyte.

6. The device according to any of claims 1-5, further **characterized by**
G) an element to generate a magnetic field that is integrated downstream from the magnetic sensor element in the vicinity of the channel structure to capture unbound particles.

7. The device according to any of claims 1-6, further **characterized by**
H) a means for sample pre-treatment that is integrated upstream from the magnetic sensor element.

8. The device according to any of claims 1-7, further **characterized by**
I) a reference channel structure including an integrated magnetic sensor element coated with the second receptor, and a defined amount of analyte is bound or not bound to magnetic particles sufficient to verify the kinetics of either a direct or a competitive binding.

9. The device according to any of claims 1-8, further **characterized by**
J) a means for identification or data storage of information about the test to be run.

10. An instrument for detecting an analyte directly or indirectly bound to one or more magnetic particle labels, the instrument comprising the device of any of claims 1-9, and one or more means selected from
a) a means to mechanically, electrically and fluidically connect the instrument to the device,
b) a means to control fluidic flow in the device,
c) a means to control and/or record generation of a variable magnetic field,
d) a means to concentrate a magnetic field in the vicinity of the magnetic sensor element, which means may be part of the instrument or the device,
e) a means to operate and read-out the magnetic sensor element,
f) a means to read out the identification or data storage of the device according to claim 9 and applying this information to configure its setup or way of operation,
g) a means to evaluate and/or record magnetic sensor element signals,
h) a means to calculate and/or record analyte concentrations from these signals,
i) a means to present analysis results to a user,
j) a means to input user commands,
k) a means to transfer data by interfaces,
l) a means to be operated by battery or line connector,
m) a magnetic field generating element placed downstream from the magnetic sensor element(s), either inside or outside the device, the element being suitable for washing by bidirectional pumping,
n) an electromagnetic means that is temporarily operated over the sensor or a permanent magnet that is temporarily moved over the sensor, both being useful for lifting unbound magnetic particles from the sensor surface(s), i.e. magnetic washing.

11. Method for quantitative and qualitative measurement of an analyte directly or indirectly bound to one or more magnetic particle labels comprising
introducing of the sample into the device according to any of claims 1-9,
optionally inserting said device into an instrument according to claim 10, and
detecting the magnetic particles bound to the magnetic sensor.

12. Method for producing a device according to any of claims 1 - 9, comprising
providing at least two injection moulded or embossed polymer plates,
b) either directly generating a magnetic sensor element in at least one channel wall or integrating a discrete magnetic sensor element in at least one channel wall,
c) introducing electrical conductors to contact said magnetic sensor element, and
d) joining the at least two injection moulded or embossed polymer plates to form a fluidic channel structure in the device.

13. Method according to claim 12, comprising
directly generating the magnetic sensor element on the polymer material in the channel structure of the device by either thin-film deposition or printing of sensing polymer materials preferably polymer electronic materials or polymer nanocomposites.

14. Use of the device according to any of claims 1-9, the instrument according to claim 10 or the method according to claim 11 for the detection and measurement of organic or inorganic analytes.

## Patentansprüche

1. Vorrichtung zum Nachweis eines Analyten, der direkt oder indirekt an ein oder mehrere Magnetpartikel-Label gebunden ist, dadurch charakterisiert, dass
A) zumindest eine Fließkanalstruktur zum Transport und Verarbeiten der Magnetpartikel und
B) ein integriertes magnetisches Sensorelement vorliegen,
wobei
das magnetische Sensorelement Teil von zumindest einer Kanalwand ist, die Fließkanalstruktur geometrisch optimiert ist für schnelles und einheitliches Binden der magnetischen Partikel/Analyten-Komplexe auf der Sensorelementoberfläche durch Leiten eines flüssigen Flusses über die Sensorelementoberfläche, wobei die magnetischen Partikel/Analyten-Komplexe auf die Oberfläche des Sensorelements gelenkt werden
durch
E1) eine Änderung des flüssigen Fließprofils, wie z.B. eine graduelle Reduktion der Kanalhöhe (Fig. 4a.1) über dem Sensor, oder
E2) durch Trägheitskräfte erzeugt durch Lenken des Flusses in einen gekrümmten Kanal (Fig. 3b.3), wobei das Sensorelement in einer Art und Weise positioniert ist, dass es durch den Fluss getroffen wird, z.B. in der äußeren Kanalwand in der Kurve,
und das magnetische Sensorelement ein GMR, TMR, CMR, EMR oder Hall-Sensor ist.

2. Die Vorrichtung gemäß Anspruch 1, desweiteren charakterisiert durch
C) ein Mittel zum Optimieren der Auflösung von magnetischen Partikeln, wobei die magnetischen Partikel, welche mit einem ersten Rezeptor und/oder Analyten
überzogen sein können oder nicht, von dem Mittel zur Optimierung der Auflösung der magnetischen Partikel freigesetzt werden.

3. Die Vorrichtung gemäß Anspruch 1 oder 2, desweiteren charakterisiert durch
D) magnetische Partikel, überzogen mit einem ersten Rezeptor, wobei die Partikel in der Vorrichtung aufbewahrt werden und in der Lage sind, an den Analyten zu binden, sobald ein Fluss der Probe durch die Kanalstruktur auftritt.

4. Die Vorrichtung gemäß irgendeinem der Ansprüche 1 bis 3, desweiteren charakterisiert durch
F) ein Element zum Vermischen der Probe und der magnetischen Partikel, um das Binden oder die Kompetition des Analyten mit dem Partikel mit Hilfe eines Rezeptors zu ermöglichen, wobei das Element zum Vermischen
F1) eine passive Struktur ist, wie z.B. eine Veränderung des Kanalquerschnitts oder der Kanalrichtung, oder eine Kombination von beiden; oder
F2) eine aktive Struktur, wie z.B. ein ferromagnetisches Element ist, das lose in der Kanalstruktur liegt oder in einer Kammer, welche bewegt werden kann durch Verändern des Magnetfeldes.

5. Die Vorrichtung gemäß irgendeinem der Ansprüche 1 bis 4, wobei die magnetische Sensoroberfläche überzogen ist mit Gold, um ein spezifisches Binden eines zweiten Rezeptors zu ermöglichen, und umgebenden Oberflächen, die nicht aus Gold sind, sondern überzogen mit einem anderen Material sind, das inert ist für das Binden an den ersten Rezeptor, an den zweiten Rezeptor oder den Analyten.

6. Die Vorrichtung gemäß irgendeinem der Ansprüche 1 bis 5, desweiteren charakterisiert durch
G) ein Element zur Erzeugung eines Magnetfeldes, das ausgehend vom magnetischen Sensorelement stromabwärts integriert ist in der Umgebung der Kanalstruktur, um ungebundene Partikel einzufangen.

7. Die Vorrichtung gemäß irgendeinem der Ansprüche 1 bis 6, desweiteren charakterisiert durch
H) ein Mittel zur Probenvorbehandlung, welches stromaufwärts von dem magnetischen Sensorelement integriert ist.

8. Die Vorrichtung gemäß irgendeinem der Ansprüche 1 bis 7, desweiteren charakterisiert durch
l) eine Referenzkanalstruktur einschließend ein integriertes magnetisches Sensorelement, überzogen mit dem zweiten Rezeptor, und einer definierten Menge an Analyt, gebunden oder nicht gebunden an Magnetpartikel, hinreichend um die Kinetik von entweder einem direkten oder kompetitiven Binden zu verifizieren.

9. Die Vorrichtung gemäß irgendeinem der Ansprüche 1 bis 8, desweiteren charakterisiert durch
J) ein Mittel zur Identifizierung oder Datenspeicherung von Information über den zu durchlaufenden Test.

10. Ein Instrument zum Nachweis eines Analyten, direkt oder indirekt gebunden an ein oder mehrere Magnetpartikel-Label, wobei das Instrument die Vorrichtung gemäß irgendeinem der Ansprüche 1 bis 9 umfasst und ein oder mehrere Mittel ausgewählt aus
a) ein Mittel zur mechanischen, elektrischen oder flüssigen Verbindung des Instruments mit der Vorrichtung
b) ein Mittel zur Steuerung des flüssigen Flusses in der Vorrichtung
c) ein Mittel zur Steuerung und/oder Aufzeichnung der Erzeugung des variablen Magnetfeldes
d) ein Mittel zur Konzentration eines Magnetfeldes in der Umgebung des Magnetsensorelements, wobei das Mittel Teil des Instruments oder der Vorrichtung sein kann
e) ein Mittel zum Betrieb und zum Auslesen des Magnetsensorelements,
f) ein Mittel zum Auslesen der Identifikation oder der Datenspeicherung der Vorrichtung gemäß Anspruch 9 und zur Anwendung dieser Information, um die entsprechende Einstellung oder Durchführungsart zu konfigurieren,
g) ein Mittel zur Bewertung und/oder Aufzeichnung von magnetischen Sensorelementsignalen,
h) ein Mittel zur Berechnung und/oder Aufzeichnung der Analytkonzentrationen aus diesen Signalen,
i) ein Mittel zur Darstellung der Analyseergebnisse für die Anwender
j) ein Mittel zur Eingabe von Anwenderbefehlen,
k) ein Mittel zur Datenübertragung durch Schnittstellen,
l) ein Mittel zur Durchführung mit Hilfe von Batterie- oder Stromversorgungseinheit,
m) ein Magnetfeld-erzeugendes Element, stromabwärts vom Sensorelement (von den Sensorelementen) platziert, entweder innerhalb oder außerhalb der Vorrichtung, wobei das Element geeignet ist zum Waschen durch bidirektionales Pumpen,
n) ein elektromagnetisches Mittel, das zeitweise betrieben wird über dem Sensor, oder ein Permanentmagnet, der zeitweise über dem Sensor bewegt wird, wobei beide bedeutsam sind für das Abheben von ungebundenen magnetischen Partikeln von der Sensoroberfläche (den Sensoroberflächen), d.h. magnetisches Waschen.

11. Ein Verfahren zur quantitativen und qualitativen Messung eines Analyten, direkt oder indirekt gebunden an ein oder mehrere Magnetpartikellabel, umfassend
Einbringen der Probe in die Vorrichtung gemäß irgendeinem der Ansprüche 1 bis 9, optionales Einbringen von besagter Vorrichtung in ein Instrument gemäß Anspruch 10, und
Nachweis der Magnetpartikel, gebunden an den Magnetsensor.

12. Verfahren zum Nachweis einer Vorrichtung gemäß irgendeinem der Ansprüche 1 bis 9, umfassend das Bereitstellen von zumindest zwei durch Spritzguss oder Stanzen erhaltene Polymerplatten,
b) entweder dieses Erzeugen eines magnetischen Sensorelements in zumindest einer Kanalwand, oder Einbringen eines abgetrennten magnetischen Sensorelements in zumindest eine Kanalwand,
c) Einbringen eines elektrischen Leiters, um einen Kontakt zu besagtem magnetischen Sensorelement herzustellen und
d) Verbinden der zumindest zwei Spritzguss-erzeugten oder per Stanzen erzeugten Polymerplatten, um eine Fließkanalstruktur in der Vorrichtung auszubilden.

13. Verfahren gemäß Anspruch 12, umfassend
das direkte Erzeugen des Magnetsensorelements auf dem Polymermaterial in der Kanalstruktur der Vorrichtung durch entweder Dünnfilmabscheidung oder Drucken von Sensorpolymermaterialien, vorzugsweise von elektronischen Polymermaterialien oder Polymernanozusammensetzungen.

14. Verwendung der Vorrichtung gemäß irgendeinem der Ansprüche 1 bis 9, des Instruments gemäß Anspruch 10 oder des Verfahrens gemäß Anspruch 11 zum Nachweis und zur Messung von organischen oder anorganischen Analyten.

## Revendications

1. Dispositif de détection d'un analyte lié directement ou indirectement à une ou plusieurs particules magnétiques de marquage, **caractérisé par**
A) au moins une structure de canal fluidique destinée à transporter et traiter des particules magnétiques, et
B) un élément de détection magnétique intégré,
où
l'élément de détection magnétique fait partie d'au moins une paroi de canal,
la géométrie de structure du canal fluidique est optimisée pour une liaison rapide et uniforme de complexes particule magnétique-analyte sur la surface de l'élément de détection, en amenant un fluide à s'écouler sur la surface de l'élément de détection, les complexes particule magnétique-analyte étant guidés vers et sur la surface de l'élément de détection magnétique
par
E1) une altération du profil d'écoulement fluidique, par exemple par une réduction graduelle de la hauteur de canal (fig 4a.1) au-dessus de l'élément de détection ou
E2) des forces d'inertie générées en guidant l'écoulement dans un canal incurvé (fig 4b.3), l'élément de détection étant positionné de façon à pouvoir être ciblé par l'écoulement, par exemple dans la paroi extérieure de canal dans la courbure
et l'élément de détection magnétique est un capteur GMR, TMR, CMR, EMR ou à effet Hall.

2. Dispositif selon la revendication 1, également **caractérisé par**
C) un moyen d'optimisation de la dissolution de particules magnétiques, tel que les particules magnétiques, qui peuvent être ou non enrobées d'un premier récepteur et/ou analyte, sont libérées à partir du moyen d'optimisation de dissolution des particules magnétiques.

3. Élément selon la revendication 1 ou 2, également **caractérisé par**
D) des particules magnétiques enrobées d'un premier récepteur, lesdites particules étant stockées dans le dispositif et pouvant se lier à l'analyte lorsqu'un écoulement d'un échantillon se produit dans la structure de canal.

4. Dispositif selon l'une quelconque des revendications 1 à 3, également **caractérisé par**
F) un élément de mélange d'échantillon et de particules magnétiques afin de faciliter la liaison ou la compétition d'un analyte à une particule au moyen d'un récepteur, l'élément de mélange étant
F1) une structure passive telle qu'une modification de section de canal ou de direction du canal, ou les deux ; ou
F2) une structure active telle qu'un élément ferromagnétique placé de manière à pouvoir bouger dans la structure de canal ou dans une chambre qui peut être mise en mouvement par un champ magnétique alternatif.

5. Dispositif selon l'une quelconque des revendications 1 à 4, la surface de détection magnétique étant revêtue d'or afin de permettre la liaison spécifique d'un second récepteur et les surfaces environnantes n'étant pas du second récepteur et de l'or mais étant revêtues d'un matériau différent qui est inerte pour ne pas se lier au premier récepteur, au second récepteur ou à l'analyte.

6. Dispositif selon l'une quelconque des revendications 1 à 5, également **caractérisé par**
G) un élément chargé de générer un champ magnétique qui est intégré en aval de l'élément de détection magnétique à proximité de la structure de canal afin de capturer des particules non liées.

7. Dispositif selon l'une quelconque des revendications 1 à 6, également **caractérisé par**
H) un moyen de pré-traitement des échantillons qui est intégré en amont de l'élément de détection magnétique.

8. Dispositif selon l'une quelconque des revendications 1 à 7, également **caractérisé par**
l) une structure de canal de référence comprenant un élément de détection magnétique revêtu du second récepteur, une quantité définie d'analyte étant liée ou non liée à des particules magnétiques de façon suffisante pour permettre de vérifier la cinétique d'une liaison directe ou compétitive.

9. Dispositif selon l'une quelconque des revendications 1 à 8, également **caractérisé par**
J) un moyen d'identification ou de stockage de données d'informations relatives à l'essai qui doit être effectué.

10. Instrument de détection d'un analyte lié directement ou indirectement à une ou plusieurs particules magnétiques de marquage, l'instrument comprenant le dispositif selon l'une quelconque des revendications 1 à 9, ainsi qu'un ou plusieurs moyens choisis parmi
a) un moyen pour connecter mécaniquement, électriquement et fluidiquement l'instrument au dispositif,
b) un moyen pour commander le débit dans le dispositif,
c) un moyen pour commander et/ou enregistrer la génération d'un champ magnétique variable,
d) un moyen pour concentrer un champ magnétique au voisinage de l'élément de détection magnétique, ledit moyen pouvant faire partie de l'istrument ou du dispositif,
e) un moyen pour actionner l'élément de détection magnétique et lire ses données,
f) un moyen de lire l'identification ou le stockage de données du dispositif selon la revendication 9 et utiliser ces informations pour configurer son paramétrage ou son mode de fonctionnement,
g) un moyen pour lire et/ou enregistrer des signaux de l'élément de détection magnétique,
h) un moyen pour calculer et/ou enregistrer des concentrations d'analyte à partir de ces signaux,
i) un moyen pour présenter des résultats d'analyse à un utilisateur,
j) un moyen pour entrer des commandes utilisateur,
k) un moyen pour transférer des données par le biais d'interfaces,
l) un moyen pour un actionnement par batterie ou par connexion à l'alimentation secteur,
m) un élément de génération de champ magnétique placé en aval du ou des éléments de détection magnétiques, à l'intérieur ou à l'extérieur du dispositif, l'élément pouvant être nettoyé par pompage bidirectionnel,
n) un moyen électromagnétique actionné temporairement au-dessus du capteur ou un aimant permanent déplacé temporairement au-dessus du capteur, les deux permettant de soulever de la ou des surfaces de l'élément de détection les particules magnétiques qui ne sont plus liées, c'est-à-dire effectuer un nettoyage magnétique.

11. Procédé de mesurage quantitatif et qualitatif d'un analyte lié directement ou indirectement à une ou plusieurs particules magnétiques de marquage consistant à
introduire l'échantillon dans le dispositif selon l'une des revendications 1 à 9,
éventuellement insérer ledit dispositif dans un instrument selon la revendication 10, et
détecter les particules magnétiques liées à l'élément de détection magnétique.

12. Procédé de production d'un dispositif selon l'une quelconque des revendications 1 à 9, consistant à
fournir au moins deux plaques polymères gaufrées ou moulées par injection,
b) produire directement un élément de détection magnétique dans au moins une paroi de canal ou intégrer un élément de détection magnétique séparé dans au moins une paroi de canal,
c) introduire des conducteurs électriques pour les mettre en contact avec ledit élément de détection magnétique, et
d) relier les au moins deux plaques polymères gaufrées ou moulées par injection afin de former une structure de canal fluidique dans le dispositif.

13. Procédé selon la revendication 12, consistant à
produire directement l'élément de détection magnétique sur le matériau polymère dans la structure de canal du dispositif, par dépôt en film mince ou impression de matériaux de détection à base de polymère, de préférence des matériaux électroniques à base de polymère ou des nanocomposites à base de polymère.

14. Utilisation du dispositif selon l'une quelconque des revendications 1 à 9, de l'instrument selon la revendication 10 ou du procédé selon la revendication 11 pour détecter et mesurer des analytes organiques ou inorganiques.
